# EUROPEAN PATENT APPLICATION

(11) **EP 2 366 695 A1**
(43) Date of publication of application: **21.09.2011**
(21) Application number: 09460048.3
(22) Date of filing: 12.11.2009
(51) Int. Cl.: C07D 235/14, C07D 403/12, A61K 31/4184, A61P 35/00

(54) **A compound, a process for its preparation, a pharmaceutical composition, use of a compound, a method for modulating or regulating serine/threonine kinases and a serine/threonine kinases modulating agent**

(71) Applicant: SELVITA S.A., 30818 Krakow (PL)
(72) Inventor: Brzozka, Krysztof, 98-200 Sieradz (PL); Czardybon, Wojciech, 43-190 Mikolow (PL); Zarebski, Adrian, 28-100 Busko-Zdroj (PL); Beuzen, Nicolas, 31-316 Krakow (PL)
(74) Representative: Maiwald Patentanwalts GmbH

(57) **Abstract**

The subject of the inventions are a compound, a process for its preparation, a pharmaceutical composition, use of a compound, a method for modulating or regulating serine/threonine kinases and a serine/threonine kinases modulating agent. The present invention relates to novel small-molecule compounds with kinase inhibitory activity, having superior properties as pharmaceutical agents, production method thereof and uses thereof. In particular, this invention relates to new derivatives of tetrabromobenzimidazole with serine/threonine kinases inhibitory properties, preferably selected from the group of Pim, HIPK, DYRK and CLK kinases, which exhibits superior pharmacological actions, and can be useful for the treatment of disease conditions, especially cancers depending on serine/threonine kinases, such as leukemias and prostate cancer.

## Description

The subject of the inventions are a compound, a process for its preparation, a pharmaceutical composition, use of a compound, a method for modulating or regulating serine/threonine kinases and a serine/threonine kinases modulating agent. The present invention relates to novel small-molecule compounds with kinase inhibitory activity, having superior properties as pharmaceutical agents, production method thereof and uses thereof. In particular, this invention relates to new derivatives of tetrabromobenzimidazole with serine/threonine kinases inhibitory properties, preferably selected from the group of Pim, HIPK, DYRK and CLK kinases, which exhibits superior pharmacological actions, and can be useful for the treatment of disease conditions, especially cancers depending on serine/threonine kinases, such as leukemias and prostate cancer.

Kinases are enzymes that modify other proteins by chemically adding phosphate groups to them (a process called phosphorylation). Phosphorylation of the targeted proteins results in a functional change of their activity but also can modify association with other proteins, trafficking and subcellular localization. It is estimated that up to 30% of all proteins can be modified by kinases. For this reason kinases are key regulators of majority of cellular pathways, especially those involved in signal transduction. Kinases are currently one of the most interesting and most extensively investigated drug targets. Among the new kinase targets for therapeutic inhibition pursued currently, Pim kinases are definitely one of the most interesting emerging molecular targets. The Pim family of serine-threonine kinases is composed of three highly homologous proteins Pim-1, -2 and -3 which play an important role in intracellular signaling and contribute to pathways involved in cell survival, inflammation, cell movement and stress response.

Around 15 years ago, the *pim-1* gene was identified as a proviral insertion site of the Moloney Murine Leukemia Virus (MoMuLV) in experiments designed to find new genes, which are involved in tumorogenesis. These findings established *pim-1* as a proto-oncogene and important player in the process of malignant transformation (Nagarajan *et al.,* 1986). In humans, the *pim-1* gene is located on chromosome 6p21.1-p21.31 (Zakut-Houri *et al.,* 1987). The *pim-1* gene comprises approximately 5 kb, contains six exons and five introns. Its cDNA contains an open reading frame of 313 codons with 94% homology to the mouse counterpart. The RNA transcript is 2.9 kilobases (kb) long (Saris *et al.,* 1991). Shortly after discovery of the gene, Pim-1 protein was identified to be a serine/ threonine kinase, belonging to CAMK kinases (group of calcium/calmodulin-regulated kinases). (Saris *et al.,* 1991; Reeves *et al.,* 1990). It contains ATP-anchor, kinase domain and an active site. There are two forms of Pim-1 in human and mouse, associated with alternative initiation codon (44kDa and 33kDa). Both forms have relatively short half-life, however the 44kDa isoform seems to be more stable. Activity of Pim-1 kinase is found in the cytoplasm, nuclear fraction and the membrane of the cells. Two isoforms have different subcellular localization - 44kDa is present mainly on the plasma membrane; 33kDa in nucleus and cytoplasm. The crystal structure of Pim-1 obtained in 2005 revealed that it is a constitutively active kinase and that in contrast to many other kinases, like Akt or MAPK kinases, Pim-1 does not require additional phosphorylation for its kinase activity, however phosphorylation of this protein may contribute to its stability (Qian *et al.,* 2005a; Qian *et al.,* 2005b). Obtained crystal structure allowed also discovering unusual structural features of the Pim-1 kinase domain. Most notably, the hinge region presents two features of particular interest: an insertion residue as well as a proline residue (Prol23), which combine to form an ATP- and inhibitor-binding region quite distinct from other protein kinases. A substrate recognition sequence of the Pim-1 kinase was identified by selective peptide mapping ((K/R)3-X-S/T-X or R/K-R/K-R-R/K-X-S/T-X, where X is an amino acid (aa) residue with a small side chain but neither basic nor acidic) (Peng *et al.,* 2007).

With regard to molecular mechanisms of Pim-1 involvement in oncogenic transformation and cancer development, one can point out several processes that are regulated by the Pim-1 kinase like stimulation of cell cycle progression, co-activation of mTOR pathway, inhibition of apoptosis, transcriptional coactivation of c-Myc, promotion of drug resistance and cell migration and metastasis Pim kinases overexpression has been reported in a variety of cancer types, ranging from hematopoietic malignancies such as diffuse B cell lymphoma, chronic lymphocytic leukemia and acute myelogenous leukemia to solid tumors such as prostate and pancreatic cancer. Acquisition of mutations in the *pim-1* gene can be one of the molecular mechanisms involved in histological transformation of follicular lymphoma (FL) and B-chronic lymphocytic leukemia (B-CLL) to diffuse large B-cell lymphoma (DLBCL)(Rossi *et al.,* 2006). Mutations of the *pim-1* gene have also been detected in cases of AIDS-associated non-Hodgkin lymphoma (Gaidano *et al.,* 2003), HCV-infected B-cell NHL patients (Libra *et al.,* 2005), primary central nervous system lymphomas (PCNSLs) (*Montesinos-Rongen et al.,* 2004), extranodal DLBCL cases and primary cutaneous marginal zone B-cell lymphoma (PCMZL) (Deutsch *et al.,* 2009; Deutsch *et al.,* 2007), primary mediastinal large B-cell lymphoma (PMLBCL)(Martelli *et al.,* 2008). Pim-1 kinase is upregulated in Epstein Barr virus infected B-cells where it enhances transcriptional activity of EBNA2 protein, essential for the growth transformation and immortalization of infected B-cells. This mechanism of action of Pim-1 kinases may predispose immortalized B-cell to undergo malignant transformation (Rainio *et al.,* 2005). Initial study performed by Amson in 1989 showed that the 33kDa isoform of Pim-1 kinase was overexpressed in approximately 30% of the analyzed samples (out of 70 hematopoietic malignancies analyzed - 51 patients and 19 cell lines), pointing toward the role of this kinase in development of myeloid and lymphoid acute leukemias (Amson *et al.,* 1989). This finding was further confirmed in a variety of other studies showing elevated levels of Pim-1 kinase in various human clinical leukemias and myeloid and lymphocytic cell lines (Meeker *et al.,* 1990). For example, Pim-1 kinase was implicated in leukemogenesis and aberrant expression levels of this kinase can be involved neoplastic transformation by phosphorylation and activation of Runx transcription factors (Aho *et al.,* 2006; Kim *et al.,* 2008). Chromosome translocations and point mutations are well-documented and frequent genetic alterations in RUNX leukemias (Penther *et al.,* 2002; Osato and Ito, 2005). Pim-1 seems to play also a crucial role in development of acute myeloid leukemias (AML). Several reports pointed out a role of Pim-1 kinase in downstream signaling by FLT3 (Fms-like tyrosine kinase 3) kinase. Constitutively activating internal tandem duplication (ITD) mutations of the receptor tyrosine kinase FLT3 play an important role in leukemogenesis, and their presence is associated with poor prognosis in AML. Constitutive FLT3 signaling upregulates Pim-1 levels in leukemia cells and the juxtamembrane domain of FLT3 is a critical domain required for this upregulation (Kim *et al.,* 2005; Vu *et al.,* 2009). Interestingly, this downstream signaling seems to be independent of STATS, Akt and MAPK signaling. Up-regulation of Pim-1 kinase contributes to the proliferative and antiapoptotic pathways induced by FLT3 signaling, and the major antiapoptotic mechanism of action is Pim-1 dependent Bad phosphorylation (Kim *et al.,* 2006). Similarly to FLT3, Pim-1 kinase is also upregulated by the Bcr-Abl fusion protein, a major cause of the chronic myelogenous leukemia. A SH3 /SH2 mediated interaction of Bcr/Abl kinase with Hck Kinase (hematopoietic cell kinase) lead to activation of Hck and phosphorylation of STATSB on the critical Tyr699 residue. Activated STATSB stimulates expression of downstream effectors like Pim-1 kinase and the A1 protein, key factors essential for in vitro transformation and in vivo leukemogenesis mediated by Bcr/Abl. (Klejman *et al.,* 2002; Nieborowska-Skorska *et al.,* 2002). Whereas inhibition of Pim-1 seems not to be sufficient to overcome Bcr/Abl mediated transformation in cancer cells, an elegant study by Adam *et al.,* showed that Pim-1 and Pim-2 play here redundant roles and simultaneous targeting of the two kinases may be an exciting therapeutic alternative to overcome resistance against small-molecule tyrosine kinase inhibitors (Nosaka and Kitamura, 2002; Adam *et al.,* 2006). Involvement of Pim-1 kinase in development of prostate cancer has been extensively studied over the past years and provided several examples of clinical importance and rationale for therapeutic indication. Already in 2001 in a microarrays screen Pim-1 expression was shown to correlate with clinical outcome of the disease and was suggested to be a better marker than the standard diagnostic test for PSA levels in serum (Dhanasekaran *et al.,* 2001). This was further confirmed in studies performed by other groups (Cibull *et al.,* 2006; Xu *et al.,* 2005; Thompson *et al.,* 2003; Valdman *et al.,* 2004). Overexpression of Pim-1 in human prostate cancer cells induces genomic instability by subverting the mitotic spindle checkpoint, centrosome amplification, chromosome misaggregation and polyploidy. When the Pim-1kinase is overexpressed in immortalized, non-tumorigenic human cells, these cells became tumorigenic (Roh *et al.,* 2008; Roh *et al.,* 2003). A very interesting finding by Zemskova and colleagues support additionally use of Pim-1 kinase inhibitors in prostate cancer treatment. Surprisingly, treatment of prostate cancer cells with docetaxel, a standard of care induces STAT3 phosphorylation and transcriptional upregulation of the *pim-1* gene. Expression of Pim-1 kinase was crucial for survival of these cells after docetaxel treatment, as shown by knock down and inhibitor experiments. This data supports further testing of novel, small molecule kinase inhibitors in combination therapies with patients with docetaxel resistance (Zemskova *et al.,* 2008). In an extensive study by Beier *et al.,* immunohistochemistry experiment performed on cells compared to non-neoplastic tissue showed overexpression of the Pim-1 protein in 98% (41/42) of invasive head and neck squamous cell carcinomas (HNSCC). This study was repeated using primary tumors and metastasis biopsies showing nearly significant correlation of Pim-1 expression with histological tumor, underlining role of Pim-1 in HNSCC developments (Beier *et al.,* 2007). In line with this finding, moderate or high expression of Pim-1 and nuclear localization was also linked to prediction of radiation response in squamocellular cancers of head and neck (Peltola *et al.,* 2009).

Pim-2 is a second member of the Pim kinase family. Functionally, it has been noticed that Pim-2 overlaps with the Akt/mTOR pathway, but is regulated independently. Both Pim-2 and Akt1 kinase regulate NFκB-dependent transcription by phosphorylation of the Cot kinase (Kane *et al.,* 2002; Hammerman *et al.,* 2004). It has been indicated that Pim-2 expression maintains high levels of NF-κB activity and NF-κB activation by Pim-2 is required for its antiapoptotic function. Moreover, the data has suggested that Cot-dependent activation of NFκB can occur via the transcriptional induction of Pim-2 rather than as a direct result of a receptor-initiated kinase cascade. Several reports showed that Pim-2 can to some extent substitute or cooperate with Pim-1 in driving tumorigenesis. As both kinases share some of the targets, like the Bad protein, they act both as prosurvival kinases preventing induction of apoptosis (Yan *et al.,* 2003; Aho *et al.,* 2004). As both Pim-1 and 2 are transcriptionally induced by upstream signaling (like FLT3 or Bcr-Abl signaling), they can cooperate and are essential in neoplastic transformation of B-cells by v-Abl oncogene (Chen *et al.,* 2008). Similarly to Pim-1, coexpression of Pim-2 and c-Myc transgene induces malignant transformation (Allen *et al.,* 1997). Also the effect on the cell cycle inhibition for both Pim-1 and Pim-2 seem to synergize in accelerating cell proliferation and cell cycle progression as shown in the literature, although the molecular mechanism of cell cycle regulation are described in detail only for Pim-1 kinase (Dai *et al.,* 2005; Chen *et al.,* 2005) There seem however also to be differences between the two kinases. Whereas recent publications on hypoxia point out its emerging role in solid tumor formation and chemoresistance, no similar reports are known for Pim-2 kinase and this role needs to be explored. On the other hand, in the recent publication by Tamburini, a special emphasis was put on the role of Pim-2 in phosphorylation of crucial 4EBP1 transcription factor (on serine S65)(Tamburini *et al.,* 2009). As shown in this publication, expression of Pim-1 in clinical samples did not correlate with the above finding, providing a proof for non-overlapping role of Pim-1 and Pim-2 in regulation of 4EBP1 phosphorylation, regulation of protein synthesis and promotion of neoplastic transformation. Similar finding were already reported in by Fox and colleagues, stressing out a crucial role of Pim-2 kinase in controlling translation independently from the Akt/mTOR pathway and pointing towards inhibition of Pim-1 kinase as an attractive option for development of new therapies, especially in acute myelogenous leukemia (Fox *et al.,* 2003).

Similarly to Pim-1, overexpression of Pim-2 has been documented in several human tumors types. One of the distinguishing reports is involvement of Pim-2 in tumorigenesis of hepatocellular carcinoma (HCC) (Gong *et al.,* 2008). Pim-2 gene expression and its protein levels were investigated in human liver cancer tissues and HepG2 cells (human hepatocellular liver carcinoma cell line). In both cases the expression of Pim-2 gene and protein was higher than in immortalized liver cell line L02, indicating its role as a tumor biomarker. Further experiments indicated that Pim-2 expression and its kinase activity are IL-3 dependent; however its apoptotic inhibition role is IL-3-inedependent. It was also found that protection against apoptosis by Pim-2 is glucose-dependent, so liver cells growing *in vivo,* surrounded by high glucose and growth factors concentration have favorable conditions to express Pim-2, however Pim-2 was unable to prevent apoptosis upon glucose deprivation. So once overexpressed in hepatic cells Pim-2 can be an important factor in tumorigenesis.

Pim-3 (also known as Kid-1 - kinase induced by depolarization) is the third member of the Pim kinase family. Similarly to Pim-2 and Pim-1, Pim-3 acts in a prosurvival way preventing apoptosis by phosphorylation of Bad. However, in contrast to Pim-1/2, Pim-3 seems to be less specific to Ser112 residue, preferably phosphorylating Ser136, Ser155 and Ser170 (Macdonald *et al.,* 2006). Pim-3 was the most effective kinase in phosphorylating Ser136 residue, which seems to be crucial for subsequent phosphorylation steps and interaction with the anti-apoptotic Bcl-XL protein. Pim phosphorylation of Bad was therefore found to promote the 14-3-3 binding and inhibition of Bcl-XL binding. Similarly to Pim-1, Pim-3 seems to be also involved in promoting vessel formation and angiogenesis (Zippo *et al.,* 2004; Zhang *et al.,* 2009b). Angiogenesis is a physiological process involving the growth of new blood vessels from pre-existing vessels. This feature play significant role in tumorigenesis because angiogenesis usually precede metastasis. Although angiogenesis is a normal process in growth and development it is also a fundamental step in the transition of tumors from a dormant state to a malignant one. It was found that Pim-3 is highly expressed both at mRNA and protein levels in endothelial cells and the protein is co-localized at the cellular lamelliopodia focal kinase (FAK), a kinase involved in cellular adhesion and spreading processes. FAK is typically located at structures known as focal adhesions; these are multi-protein structures that link the extracellular matrix to the cytoplasmic cytoskeleton. It is recruited as a participant in focal adhesion dynamics between cells and has a role in motility and cell survival. FAK have also tyrosine kinase activity and originally identified as a substrate for the oncogene protein. After treatment with cytochalasin D which disrupts actin microfilaments, Pim-3 was dispersed from lamelliopodia suggesting strong interaction of Pim-3 with cytoskeleton. Furthermore knockdown of Pim-3 by siRNA had significant effects on endothelial cells migration, proliferation and formation of sprouts. In light of this finding Pim-3 kinase seems to be a new and promising target for novel inhibitors of angiogenesis.

Pim-3 overexpression has been observed in several human cancers, mainly solid tumors like gastrointestinal, colon or liver cancers where expression of Pim-3 seems to be also a poor prognostic marker, however its role in development of pancreatic adenocarcinoma has been studies in more detail (Popivanova *et al.,* 2007; Zheng *et al.,* 2008). Pim-3 was found to be expressed in malignant lesions of the pancreas but not in normal pancreatic tissue (Li *et al.,* 2006). In line with this finding, Pim-3 mRNA and protein were constitutively expressed in all examined human pancreatic cancer cell lines. Knock down of the *pim-1* mRNA levels resulted in apoptosis of the cells, proving essential role of Pim-3 in inhibition of apoptosis in pancreatic cancer cell lines. Further experiments showed that expression of Pim-3 in pancreatic cell lines is controlled by binding of the Ets-1 protein to the 5'-flanking region of human *pim-3* gene between -249 and -183 bp (Li *et al.,* 2009). Overexpression of Ets-1 transcription factor was able to stimulate transcription and translation of the Pim-3 kinase. These observations indicate that the transcription factor Ets-1 can induce aberrant Pim-3 expression and subsequently prevent apoptosis in human pancreatic cancer cells. Despite the fact that Pim-3 is a kinase of emerging role in cancer development, presented above results implicate how important and diversified roles Pim-3 may play in tumorigenesis and provide rationale for further development of Pim-3 inhibitors for cancer treatment.

CLK kinases belong to Lammer dual specificity kinase subfamily and phosphorylate serines, threonines and tyrosines. The family consists of 4 members (Clk1/Sty and Clk2-4). CLKs are dual-specificity kinases, which have the ability to autophosphorylate themselves at tyrosine residues but phosphorylate their substrates exclusively on serine/threonine residues. These kinases phosphorylate serine- and arginine-rich (SR) proteins of the spliceosomal complex like ASF/SF2, SRp40 and SRp55, critical components of splicesosomes (Soret and Tazi, 2003; Stojdl and Bell, 1999). Alternative splicing is a crucial mechanism for generating protein diversity. Different splice variants of a given protein can display different and even antagonistic biological functions. Therefore, appropriate control of their synthesis is required to assure the complex orchestration of cellular processes within multicellular organisms. Mechanisms that alter the accuracy of either constitutive or alternative splicing could have a profound impact on human pathogenesis, in particular in tumor development and progression (Hagiwara, 2005).

Clk kinases were so far shown to be implicated in regulation of alternative splicing of only few genes like tissue factor, VEGF receptor and PKCbeta II kinase. Apart from VEGF splicing, where Clk seem to have rather beneficial role and act in a anti-angiogenic way leading to formation of anti-angiogenic form of VEGF-b, there is no direct evidence in literature on their role in cancer development. There are however indirect reports showing that they might play a role in such cancers like erythroleukemia. In a study by Garcia-Sacristan from 2005 it was shown that Clk/STY, as well as other members of the family (clk2, clk3 and clk4), are up-regulated during HMBA-induced erythroleukemia cell differentiation(Garcia-Sacristan *et al.,* 2005). In a recent article by Jiang *et al.,* it was shown that Akt2, in response to insulin, resulted in phosphorylation of Clk/Sty, which then altered SR protein phosphorylation in concert with Akt2 (Jiang *et al.,* 2009). Apart from its importance in diabetes, the influence of Clk inhibitor on PKC beta splicing can be important in cancer treatment. There is evidence that PKCbeta can contribute in several ways to tumor formation. In addition to direct effects on tumor cells, PKCbeta is involved in tumor host mechanisms such as inflammation and angiogenesis. Elevated expression of PKCbetaII seems to be an early event in colon cancer development and transgenic overexpression of PKCbetaII in the intestine induces hyper-proliferation and an invasive phenotype in epithelial cells by activating beta-catenin/Apc signaling pathway. A study by Abrams demonstrated that overexpression of the PKCbetaII isoform is a feature of CLL (chronic lymphocytic leukemia) cells and that activity of this enzyme strongly correlates with CLL cell response to BCR engagement.

Substituted with halogens benzimidazole derivatives has been previously described as protein kinase inhibitors of IRAK, CK2, DYRK, HIPK and Pim kinases (WO03/030902 A1; WO2005/092866; Gianoncelli *et al.,* 2009; Pagano *et al.,* 2008 and 2004; Andrzejewska *et al.,* 2003).

Despite of the work cited above which supports a principle that inhibition of Pim kinases will be beneficial for treatment of various disease conditions there is still a need for potent and selective inhibitors of these kinases exists with optimized efficacy, druggability and safety profiles.

The goal of the present invention is to provide novel derivatives of tetrabromobenzimidazole with more potent anticancer activity *in vitro* in comparison to the previously published compounds and improved potency and specificity towards serine/threonine kinases. The invention also reports on CLK kinases as new kinase targets of tetrabromobenzimidazoles. Moreover, certain compounds presented in this application exert improved solubility and are therefore better candidates for pharmaceutical development and treatment of diseases like cancer and immunological disorders.

It has been found that compounds of this invention and pharmaceutically acceptable compositions thereof are effective inhibitors of the Pim kinase family, but also other kinases like the HIPK, DYRK and CLK kinases. In certain embodiments these compounds are selective Pim-1 kinase inhibitors which in contrast to previously published tetrabromoderivatives exert a much higher specificity in Pim-1 kinase inhibition over CK2 kinase. These compounds exert also improved cytotoxic activity in vitro when tested on a panel of neoplastic cell lines. In contrast to tetrabromobenzimidazoles derivatives published in the prior art, which in general did not reach the ED₅₀ values below 10 µM for more than 2 or 3 neoplastic cell lines, compounds presented in the application show better cytotoxicity defined as ED₅₀ values below 10 µM in at least five different neoplastic cell lines of both hematological and solid tumor origin. This feature will allow broader therapeutic indication of the compounds from the current application to treat various cancer types. In addition, one of the technical features that renders further pharmaceutical development and medical use of the tetrabromobenzimidazoles is their very low solubility in physiological solutions and pH above 7. Surprisingly, despite the four bromines moiety, by changes introduced in the R₁, R₂ and R₃ substituents, we were able not only to improve serine/threonine kinase inhibition and *in vitro* cytotoxicity, but also significantly to increase solubility of these compounds in pH 7.4.

The subject of invention is a compound of formula (I): 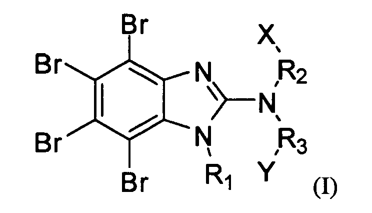 or an enantiomer thereof or a mixture of its enantiomers or its pharmaceutically acceptable salt, or pharmaceutically acceptable prodrugs or pharmaceutically active metabolites, wherein: R₁ is selected from a group consisting of: hydrogen atom, substituted or unsubstituted alkyl or cycloalkyl, substituted or unsubstituted alkenyl or alkynyl, substituted or unsubstituted aryl, substituted or unsubstituted heteroalkyl or heteroaryl;
R₂ and R₃ are independently selected from a group consisting of: hydrogen atom, substituted or unsubstituted hydrocarbon chain, substituted or unsubstituted cycloalkyl, substituted or unsubstituted aryl, substituted or unsubstituted heteroaryl, substituted or unsubstituted heterocycloalkyl;
X and Y are independently selected from a hydrogen atom, substituted or unsubstituted heteroaryl, guanidinyl , -NH₂, -NHR0 or N(R0)₂ where R0 represents a substituted or unsubstituted alkyl, aryl or heteroaryl, and wherein X and Y can also represent a substituted or unsubstituted heterocyclic moiety having 3-10 atoms wherein at least one of the ring atoms is nitrogen
or
R₂ and R₃ are connected to a substituted or unsubstituted heterocycloalkyl,

Wherein the following structures are excluded:
2-amino-4,5,6,7-tetrabromo-1H-benzimidazole
2-methylamino-4,5,6,7-tetrabromo-1H-benzimidazole
2-dimethylamino-4,5,6,7-tetrabromo-1H-benzimidazole
2-ethanolamino-4,5,6,7-tetrabromo-1H-benzimidazole
2-isopropylamino-4,5,6,7-tetrabromo-1H-benzimidazole
2-(2-hydroxypropylamino)-4,5,6,7-tetrabromo-1H-benzimidazole
2-(3-hydroxypropylamino)-4,5,6,7-tetrabromo-1H-benzimidazole
2-(2-dimethylaminoethylamino)-4,5,6,7-tetrabromo-1H-benzimidazole
2-dimethylamino-4,5,6,7-tetrabromo-1-methyl-benzimidazole
2-isopropylamino-4,5,6,7-tetrabromo-1methyl-benzimidazole
2-(methyl(4,5,6,7-tetrabromo-1-methyl-1H-benzo[d]imidazol-2-yl)amino)ethanol
(2-dimethylamino-4,5,6,7-tetrabromobenzoimidazol-1-yl)-acetic acid ethyl ester
(2-dimethylamino-4,5,6,7-tetrabromobenzoimidazol-1-yl)-acetic acid.

In a preferred embodiment of the compounds of the invention, R₁ is -H or lower alkyl, R₂ is - H, R₃ is a straight-chain alkyl, and Y is a primary amine.

The following is a list of definitions for terms used herein.
"Alkyl" is a saturated hydrocarbon chain having 1 to 15 carbon atoms, preferably 1 to 10, more preferably 1 to 4 carbon atoms. "Alkenyl" is a hydrocarbon chain having at least one (preferably only one) carbon-carbon double bond and having 2 to 15 carbon atoms, preferably 2 to 10, more preferably 2 to 4 carbon atoms. "Alkynyl" is a hydrocarbon chain having at least one (preferably only one) carbon-carbon triple bond and having 2 to 15 carbon atoms, preferably 2 to 10, more preferably 2 to 4 carbon atoms. Alkyl, alkenyl and alkynyl chains (referred to collectively as "hydrocarbon chains") may be straight or branched and may be unsubstituted or substituted. Preferred branched alkyl, alkenyl and alkynyl chains have one or two branches, preferably one branch. Preferred chains are alkyl. Alkyl, alkenyl and alkynyl hydrocarbon chains each may be unsubstituted or substituted with from 1 to 4 substituents; when substituted, preferred chains are mono-, di-, or tri-substituted. Alkyl, alkenyl and alkynyl hydrocarbon chains each may be substituted with halo, hydroxy, aryloxy (e.g. phenoxy), heteroaryloxy, acyloxy (e.g. acetoxy), carboxy, aryl (e.g. phenyl), heteroaryl, cycloalkyl, heterocycloalkyl, spirocycle, amino, amido, acylamino, keto, thioketo, cyano, or any combination thereof. Preferred hydrocarbon groups include methyl, ethyl, propyl, isopropyl, butyl, vinyl, allyl, and butenyl.

Also, as referred to herein, a "lower" alkyl, alkenyl or alkynyl moiety (e.g. "lower alkyl") is a chain comprised of 1 to 6, preferably from 1 to 4, carbon atoms in the case of alkyl and 2 to 6, preferably 2 to 4, carbon atoms in the case of alkenyl and alkynyl.

"Aryl" is an aromatic hydrocarbon ring. Aryl rings are monocyclic or fused bicyclic ring systems. Monocyclic aryl rings contain 6 carbon atoms in the ring. Monocyclic aryl rings are also referred to as phenyl rings. Bicyclic aryl rings contain from 8 to 17 carbon atoms, preferably 9 to 12 carbon atoms, in the ring. Bicyclic aryl rings include ring systems wherein one ring is aryl and the other ring is aryl, cycloalkyl, or heterocycloakyl. Preferred bicyclic aryl rings comprise 5-, 6- or 7-membered rings fused to 5-, 6-, or 7-membered rings. Aryl rings may be unsubstituted or substituted with from 1 to 4 substituents on the ring. Aryl may be substituted with halo, cyano, nitro, hydroxy, carboxy, amino, acylamino, alkyl, heteroalkyl, haloalkyl, phenyl, aryloxy, alkoxy, heteroalkyloxy, carbamyl, haloalkyl, methylenedioxy, heteroaryloxy, or any combination thereof. Preferred aryl rings include naphthyl, tolyl, xylyl, and phenyl. The most preferred aryl ring radical is phenyl.

"Cycloalkyl" is a saturated or unsaturated hydrocarbon ring. Cycloalkyl rings are not aromatic. Cycloalkyl rings are monocyclic, or are fused, spiro, or bridged bicyclic ring systems. Monocyclic cycloalkyl rings contain from about 3 to about 9 carbon atoms, preferably from 3 to 7 carbon atoms, in the ring. Bicyclic cycloalkyl rings contain from 7 to 17 carbon atoms, preferably from 7 to 12 carbon atoms, in the ring. Preferred bicyclic cycloalkyl rings comprise 4-, 5-, 6- or 7-membered rings fused to 5-, 6-, or 7-membered rings. Cycloalkyl rings may be unsubstituted or substituted with from 1 to 4 substituents on the ring. Cycloalkyl may be substituted with halo, cyano, alkyl, heteroalkyl, haloalkyl, phenyl, keto, hydroxy, carboxy, amino, acylamino, aryloxy, heteroaryloxy, or any combination thereof. Preferred cycloalkyl rings include cyclopropyl, cyclopentyl, and cyclohexyl.

"Heteroatom" is a nitrogen, sulfur, or oxygen atom. Groups containing more than one heteroatom may contain different heteroatoms.

"Heteroalkyl" is a saturated or unsaturated chain containing carbon and at least one heteroatom, wherein no two heteroatoms are adjacent. Heteroalkyl chains contain from 2 to 15 member atoms (carbon and heteroatoms) in the chain, preferably 2 to 10, more preferably 2 to 5. For example, alkoxy (i.e. -O-alkyl or -O-heteroalkyl) radicals are included in heteroalkyl. Heteroalkyl chains may be straight or branched. Preferred branched heteroalkyl have one or two branches, preferably one branch. Preferred heteroalkyl are saturated. Unsaturated heteroalkyl have one or more carbon-carbon double bonds and/or one or more carbon-carbon triple bonds. Preferred unsaturated heteroalkyls have one or two double bonds or one triple bond, more preferably one double bond. Heteroalkyl chains may be unsubstituted or substituted with from 1 to 4 substituents. Preferred substituted heteroalkyl are mono-, di-, or tri-substituted. Heteroalkyl may be substituted with lower alkyl, haloalkyl, halo, hydroxy, aryloxy, heteroaryloxy, acyloxy, carboxy, monocyclic aryl, heteroaryl, cycloalkyl, heterocycloalkyl, spirocycle, amino, acylamino, amido, keto, thioketo, cyano, or any combination thereof.

"Heteroaryl" is an aromatic ring containing carbon atoms and from 1 to about 6 heteroatoms in the ring. Heteroaryl rings are monocyclic or fused bicyclic ring systems. Monocyclic heteroaryl rings contain from about 5 to about 9 member atoms (carbon and heteroatoms), preferably 5 or 6 member atoms, in the ring. Bicyclic heteroaryl rings contain from 8 to 17 member atoms, preferably 8 to 12 member 60 atoms, in the ring. Bicyclic heteroaryl rings include ring systems wherein one ring is heteroaryl and the other ring is aryl, heteroaryl, cycloalkyl, or heterocycloalkyl. Preferred bicyclic heteroaryl ring systems comprise 5-, 6- or 7-membered rings fused to 5-, 6-, or 7-membered rings. Heteroaryl rings may be unsubstituted or substituted with from 1 to 4 substituents on the ring. Heteroaryl may be substituted with halo, cyano, nitro, hydroxyl, carboxy, amino, acylamino,alkyl, heteroalkyl, phenyl, alkoxy, aryloxy, heteroaryloxy, or any combination thereof.

Preferred heteroaryl rings include, but are not limited to, the following:

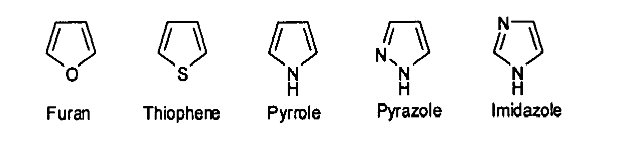

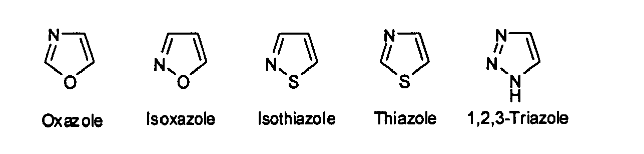

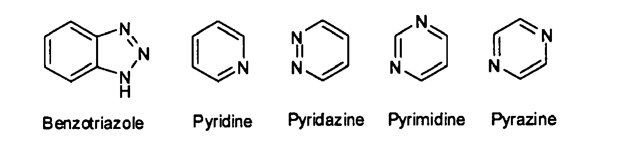

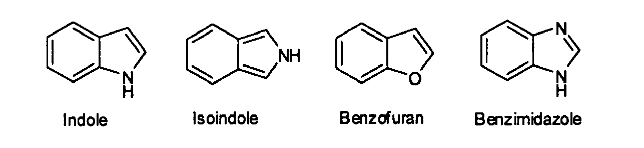

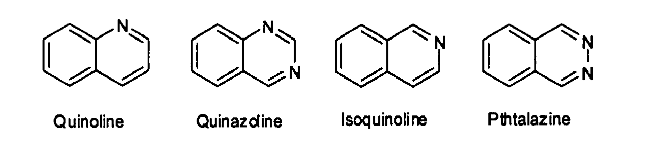

"Heterocycloalkyl" is a saturated or unsaturated ring containing carbon atoms and from 1 to about 4 (preferably 1 to 3) heteroatoms in the ring. Heterocycloalkyl rings are not aromatic. Heterocycloalkyl rings are monocyclic, or are fused, bridged, or spiro bicyclic ring systems. Monocyclic heterocycloalkyl rings contain from about 3 to about 9 member atoms (carbon and heteroatoms), preferably from 5 to 7 member atoms, in the ring. Bicyclic heterocycloalkyl rings contain from 7 to 17 member atoms, preferably 7 to 12 member atoms, in the ring. Bicyclic heterocycloalkyl rings contain from about 7 to about 17 ring atoms, preferably from 7 to 12 ring atoms. Bicyclic heterocycloalkyl rings may be fused, spiro, or bridged ring systems. Preferred bicyclic heterocycloalkyl rings comprise 5-, 6- or 7-membered rings fused to 5-, 6-, or 7-membered rings. Heterocycloalkyl rings may be unsubstituted or substituted with from 1 to 4 substituents on the ring. Heterocycloalkyl may be substituted with halo, cyano, hydroxy, carboxy, keto, thioketo, amino, acylamino, acyl, amido, alkyl, heteroalkyl, haloalkyl, phenyl, alkoxy, aryloxy or any combination thereof. Preferred substituents on heterocycloalkyl include halo and haloalkyl. Preferred heterocycloalkyl rings include, but are not limited to, the following:

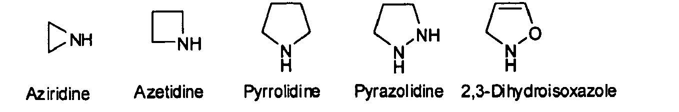

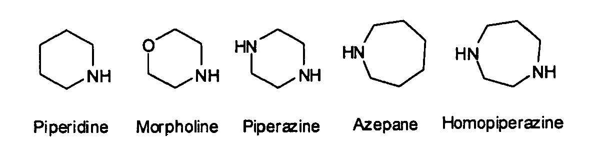

While alkyl, heteroalkyl, cycloalkyl, and heterocycloalkyl groups may be substituted with hydroxy, amino, and amido groups as stated above, the following are not envisioned in the invention:
Enols (OH attached to a carbon bearing a double bond).
Amino groups attached to a carbon bearing a double bond (except for vinylogous amides).

More than one hydroxy, amino, or amido attached to a single carbon (except where two nitrogen atoms are attached to a single carbon atom and all three atoms are member atoms within a heterocycloalkyl ring).

Hydroxy, amino, or amido attached to a carbon that also has a heteroatom attached to it. Hydroxy, amino, or amido attached to a carbon that also has a halogen attached to it.

The invention also relates to pharmaceutically acceptable prodrugs of the compounds described herein, and methods employing such pharmaceutically acceptable prodrugs. The term "prodrug" means a precursor of a designated compound that, following administration to a subject, yields the compound *in vivo* via a chemical or physiological process such as solvolysis or enzymatic cleavage, or under physiological conditions (e.g. a prodrug on being brought to physiological pH is converted to the compound of the present invention). A "pharmaceutically acceptable prodrug" is a prodrug that is non-toxic, biologically tolerable, and otherwise biologically suitable for administration to the subject. Illustrative procedures for the selection and preparation of suitable prodrug derivatives are described, for example, in "Design of Prodrugs", ed. H. Bundgaard, Elsevier, 1985.

The present invention also relates to the pharmaceutically active metabolites of the compounds of the invention, and uses of such metabolites in the methods of the invention. A "pharmaceutically active metabolite" means a pharmacologically active product of metabolism in the body of a compound of the present invention or salt thereof. Prodrugs and active metabolites of a compound may be determined using routine techniques known or available in the art.

Preferably, R₁ is selected among hydrogen atom, substituted or unsubstituted alkyl, cycloalkyl, alkenyl or alkynyl;
R₂ is H, and R₃ is a C₂-C₅ alkyl substituted with Y selected from: -NH₂, -NH(C=NH)-NH₂, - NHR0, -N(R0)₂, wherein R0 represents a substituted or unsubstituted alkyl, aryl or heteroaryl. Preferably, the compound is selected from:
N1-(4,5,6,7-tetrabromo-1-methyl-1H-benzo[d]imidazol-2-yl)ethane-1,2-diamine;
N1-(4,5,6,7-tetrabromo-1H-benzo[d]imidazol-2-yl)propane-1,3-diamine;
N1-(4,5,6,7-tetrabromo-1-methyl-1H-benzo[d]imidazol-2-yl)propane-1,3-diamine;
N1-(4,5,6,7-tetrabromo-1-isopropyl-1H-benzo[d]imidazol-2-yl)propane-1,3-diamine;
1-(2-(4,5,6,7-tetrabromo-1H-benzo[d]imidazol-2-ylamino)ethyl)guanidine;
1-(3-(4,5,6,7-tetrabromo-1H-benzo[d]imidazol-2-ylamino)propyl)guanidine;
N1,N1-dimethyl-N3-(4,5,6,7-tetrabromo-1H-benzo[d]imidazol-2-yl)propane-1,3-diamine;
N1,N1-dimethyl-N3-(4,5,6,7-tetrabromo-1-methyl-1H-benzo[d]imidazol-2-yl)propane-1,3-diamine;
N1-(4,5,6,7-tetrabromo-1H-benzo[d]imidazol-2-yl)butane-1,4-diamine;
N1-(4,5,6,7-tetrabromo-1-methyl-1H-benzo[d]imidazol-2-yl)butane-1,4-diamine;
N1-(4,5,6,7-tetrabromo-1-isopropyl-1H-benzo[d]imidazol-2-yl)butane-1,4-diamine;

Preferably, R₁ is selected among hydrogen atom, substituted or unsubstituted alkyl, cycloalkyl, alkenyl or alkynyl; and R₂ is H, and R3 is a C₂-C₅ alkyl substituted with Y selected from a substituted or unsubstituted heterocyclic moiety having 3-10 atoms, preferentially the following:

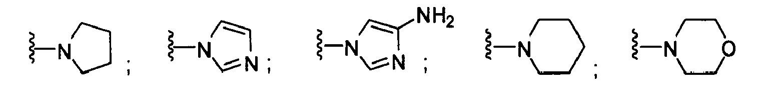

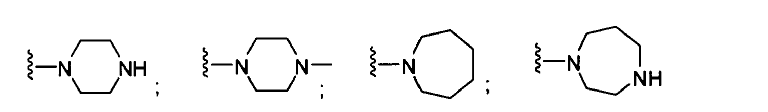

Preferably, the compound is selected from:
4,5,6,7-tetrabromo-N-(2-(pyrrolidin-1-yl)ethyl)-1H-benzo[d]imidazol-2-amine;
N-(2-(1H-imidazol-1-yl)ethyl)-4,5,6,7-tetrabromo-1H-benzo[d]imidazol-2-amine;
4,5,6,7-tetrabromo-N-(2-(piperidin-1-yl)ethyl)-1H-benzo[d]imidazol-2-amine;
4,5,6,7-tetrabromo-N-(2-morpholinoethyl)-1H-benzo[d]imidazol-2-amine;
4,5,6,7-tetrabromo-N-(2-piperazin-1-yl)ethyl)-1H-benzo[d]imidazol-2-amine;
N-(2-(azepan-1-yl)ethyl)-4,5,6,7-tetrabromo-1H-benzo[d]imidazol-2-amine;
N-(2-(azepan-1-yl)ethyl)-4,5,6,7-tetrabromo-1-methyl-1H-benzo[d]imidazol-2-amine;
N-(2-(1,4-diazepan-1-yl)ethyl)-4,5,6,7-tetrabromo-1H-benzo[d]imidazol-2-amine;
N-(2-(1,4-diazepan-1-yl)ethyl)-4,5,6,7-tetrabromo-1-methyl-1H-benzo[d]imidazol-2-amine;
N-(2-(1,4-diazepan-1-yl)ethyl)-4,5,6,7-tetrabromo-1-isopropyl-1H-benzo[d]imidazol-2-amine;
Preferably, R₁ is selected from hydrogen atom, substituted or unsubstituted alkyl, cycloalkyl, alkenyl or alkynyl; R₂ and R₃ are independently selected from C₂₋₅ alkyl optionally substituted with X and/or Y selected from: -NH₂, -NH(C=NH)-NH₂, -NHR0, -N(RO)₂, where R0 represents a substituted or unsubstituted alkyl , aryl or heteroaryl, and wherein the compound is preferably selected from:
   N1-ethyl-N1-(4,5,6,7-tetrabromo-1H-benzo[d]imidazol-2-yl)ethane-1,2-diamine;
   N1-(2-aminoethyl)-N1-(4,5,6,7-tetrabromo-1H-benzo[d]imidazol-2-yl)ethane-1,2-diamine;
   N1-(2-aminoethyl)-N1-(4,5,6,7-tetrabromo-1-methyl-1H-benzo[d]imidazol-2-yl)ethane-1,2-diamine;
   N1-ethyl-N1-(4,5,6,7-tetrabromo-1H-benzo[d]imidazol-2-yl)propane-1,3-diamine;
   N1-(3-aminopropyl)-N1-(4,5,6,7-tetrabromo-1H-benzo[d]imidazol-2-yl)propane-1,3-diamine;
   N1-(3-aminopropyl)-N3,N3-dimethyl-N1-(4,5,6,7-tetrabromo-1H-benzo[d]imidazol-2-yl)propane-1,3-diamine;
   N1-(3-aminopropyl)-N3,N3-dimethyl-N1-(4,5,6,7-tetrabromo-1-methyl-1H-benzo[d]imidazol-2-yl)propane-1,3-diamine;
   1-(3-((3-(dimethylamino)propyl)(4,5,6,7-tetrabromo-1H-benzo[d]imidazol-2-yl)amino)propyl)guanidine;
   1-(3-((3-(dimethylamino)propyl)(4,5,6,7-tetrabromo-1-methyl-1H-benzo[d]imidazol-2-yl)amino)propyl)guanidine.

Preferably, R₁ is selected from hydrogen atom, substituted or unsubstituted alkyl, cycloalkyl, alkenyl or alkynyl; R₂ is H, and R₃ is selected from substituted or unsubstituted aryl or substituted or unsubstituted heteroaryl , and wherein the compound is preferably selected from:
4,5,6,7-tetrabromo-N-phenyl-1H-benzo[d]imidazol-2-amine;
4,5,6,7-tetrabromo-l-methyl-N-phenyl-1H-benzo[d]imidazol-2-amine;
N1-(4,5,6,7-tetrabromo-1H-benzo[d]imidazol-2-yl)benzene-1,4-diamine;
N1-(4,5,6,7-tetrabromo-1-methyl-1H-benzo[d]imidazol-2-yl)benzene-1,4-diamine;
N1,N1-dimethyl-N4-(4,5,6,7-tetrabromo-1H-benzo[d]imidazol2-yl)benzene-14-diamine;
N1,N1-dimethyl-N4-(4,5,6,7-tetrabromo-1-methyl-1H-benzo[d]imidazol-2-yl)benzene-1,4-diamine.

Preferably, R₁is selected among hydrogen atom, substituted or unsubstituted alkyl, cycloalkyl, alkenyl or alkynyl, and R₂ and R₃ are connected to a substituted or unsubstituted heterocycloalkyl, and wherein the compound is preferably selected from:
4,5,6,7-tetrabromo-2-(pyrrolidin-1-yl)-1H-benzo[d]imidazole;
4,5,6,7-tetrabromo-2-(piperidin-1-yl)-1H-benzo[d]imidazole;
4-(4,5,6,7-tetrabromo-1H-benzo[d]imidazol-2-yl)morpholine;
4,5,6,7-tetrabromo-2-(piperazin-1-yl)-1H-benzo[d]imidazole;
4,5,6,7-tetrabromo-2-(4-methylpiperazin-1-yl)-1H-benzo[d] imidazole;
4,5,6,7-tetrabromo-1-methyl-2-(4-methylpiperazin-1-yl)-1H-benzo[d]imidazole;
4,5,6,7-tetrabromo-1-isopropyl-2-(4-methylpiperazin-1-yl)-1H-benzo[d]imidazole.

Preferably, a pharmaceutically acceptable salt is selected from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate and the like.

A "pharmaceutically-acceptable salt" is a cationic salt formed at any acidic (e.g. carboxylic acid) group, or an anionic salt formed at any basic (e.g. amino) group. Many such salts are known in the art, as described in World Patent Publication 87/05297, Johnston et al., published Sep. 11, 1987 incorporated by reference herein. Preferred cationic salts include the alkali metal salts (such as sodium and potassium), and alkaline earth metal salts (such as magnesium and calcium) and organic salts. Preferred anionic salts include the halides (such as chloride salts), sulfonates, carboxylates, phosphates, and the like.

Such salts are well understood by the skilled artisan, and the skilled artisan is able to prepare any number of salts given the knowledge in the art. Furthermore, it is recognized that the skilled artisan may prefer one salt over another for reasons of solubility, stability, formulation ease and the like.

The next subject of invention is a pharmaceutical composition, comprising a therapeutically effective amount of at least one compound selected from the group consisting of compounds as described above and optionally comprising a therapeutic agent selected from a chemotherapeutic or anti-proliferative agent, an immunomodulatory or immunosuppressive agent, or an anti-inflammatory agent.

The term "composition" is intended to encompass a product comprising the specified ingredients in the specified amounts, as well as any product which results, directly or indirectly, from combinations of the specified ingredients in the specified amounts. The active agents of the invention are used, alone or in combination with one or more additional active ingredients, to formulate pharmaceutical compositions of the invention. A pharmaceutical composition of the invention comprises an effective amount of at least one active agent in accordance with the invention. As known in pharmaceutical technology, at least one pharmaceutically acceptable carrier may be comprised in embodiments of pharmaceutical compositions according to this invention.

Preferably, the said composition optionally comprises a therapeutic agent selected from a chemotherapeutic or anti-proliferative agent, an immunomodulatory or immunosuppressive agent, or an anti-inflammatory agent.

Preferably, the therapeutically effective amount provided in the treatment is administered in an amount of about 0,01 to 1,000 mg/kg at least once a day for the duration of the treatment.

The term "therapeutically effective amount" as used herein, means that amount of active compound or pharmaceutical agent that elicits the biological or medicinal response in a tissue system, animal or human that is being sought by a researcher, veterinarian, medical doctor or other clinician, which includes alleviation of the symptoms of a disease or disorder. When referring to modulating the target receptor, a "therapeutically effective amount" means an amount sufficient to at least affect the activity of such receptor. Measuring the activity of the target kinase may be performed by routine analytical methods. Target kinase modulation is useful in a variety of settings, including assays. In addition, effective amounts or doses of the active agents of the present invention may be ascertained by routine methods such as modeling, dose escalation studies or clinical trials, and by taking into consideration routine factors, e.g., the mode or route of administration or drug delivery, the pharmacokinetics of the agent, the severity and course of the disease, disorder, or condition, the subject's previous or ongoing therapy, the subject's health status and response to drugs, and the judgment of the treating physician.

The amount varies according to the size, age and response pattern of the patient, the severity of the disorders, the judgment of the attending physician and the like.

Preferably, the said composition is administered parenterally, vaginally, rectally, transdermally, orally or through the otolaryngologal sphere.

Preferably, it further comprises a pharmaceutically acceptable carrier selected from the group consisting of flavoring agents, sweetener, lubricants, solubilizers, suspending agents, fillers, glidants, compression aides, binders, tablet-disintegrating agents, effervescing agent, wetting agent encapsulating materials, dyestuff, and mixtures thereof wherein carrier is chosen from solid or liquid carriers, whether sterile or not.

Preferably, the said composition optionally further comprises of one or more additional pharmaceutically acceptable carriers selected from the group consisting of a flavoring agents, sweeteners, binders, diluents, solubilizer, lubricants, suspending agents, fillers, glidants, compression aides, disintegrating agents, effervescing agents, dyestuffs, wetting agents or encapsulating materials and mixtures thereof wherein carrier is chosen from solid or liquid carriers, whether sterile or not. In powders, the carrier and compound is a finely divided solid. In tablets, said compound is mixed with a carrier having the necessary compression properties in suitable proportions and compacted in the shape and size desired. Said powders and tablets contain from 0,1 to 99% by weight of the compound. Solid carriers suitable for use in the composition of the invention include but are not limited to calcium phosphate, magnesium stearate, talc, sugars, lactose, dextrin, starch, gelatin, cellulose, methyl cellulose, sodium carboxymethyl cellulose, polyvinylpyrrolidine, low melting waxes and ion exchange resins.

Any pharmaceutically acceptable liquid carrier suitable for preparing solutions, suspensions, emulsions, syrups and elixirs can be employed in the composition of the invention. Is that case, the compound is dissolved or suspended in a pharmaceutically acceptable liquid carrier such as water, an organic solvent, or pharmaceutically acceptable oil or fat, or a mixture thereof. Said liquid composition contain additionally or not other suitable pharmaceutical additives such as solubilizers, emulsifiers, buffers, preservatives, sweeteners, flavoring agents, suspending agents, thickening agents, coloring agents, viscosity regulators, stabilizers, osmoregulators, or the like.

Examples of liquid carriers suitable for oral and parenteral administration include water, particularly containing additives as above but not limited to cellulose derivatives, preferably sodium carboxymethyl cellulose solution, alcohols, including monohydric alcohols and polyhydric alcohols, such as glycols or their derivatives, or oils such as fractionated coconut oil, cottonseed oil and arachid oil. For parenteral administration the carrier may also be an oily ester such as ethyl oleate or isopropyl myristate.

Preferably, the said composition is a sterile solution or suspension suitable for parenteral administration, including but not limited to intramuscular, intraperitoneal, intravenous, intrathecal or subcutaneous injection or perfusion.

Preferably the pharmaceutical composition is suitable for oral administration either liquid or solid composition form, including pills, tablets, capsules, sachets, dragees, powders, granules, lozenges, powders for reconstitution, orally disintegrating tablet, films, osmotic controlled release capsule, elixir, emulsion, syrup, suspension, tincture, solutions or powder for inhalation and nebulization, or sublingual administration.

Preferably, the said composition is a formulation for transdermal, rectal, vaginal administration, including ointments, creams, lotions, liniments, gels, paste, films, suppositories, enemas and pessaries.

Preferably, the said composition is a formulation for administration through the eyes, ears and nose.

Preferably, the composition is an immediate, extended or slow- release formulation.

Preferably, the therapeutically effective amount is provided in the treatment of a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis. Thus, the terms "cancer", "neoplasm" or "neoplastic," as provided herein, refer to a cell afflicted by any one of the above-identified conditions but are not limited thereto.

Preferably, it is for the prevention or treatment of neoplastic conditions, especially related with the modulation or regulation of serine/threonine kinases, preferably selected from the group of Pim, HIPK, DYRK and CLK kinases.

Serine/threonine kinases inhibitors disclosed in this application may also be used for treating immune disorders such as but not limited to bone marrow transplant rejection, rheumatoid arthritis, psoriasis, type I diabetes mellitus, multiple sclerosis. Serine/threonine kinases can be also used for treatment of infectious diseases exemplified by but not limited to infections with herpesiviruses.

The next subject of invention is a use of the compound of Formula I for the preparation of a pharmaceutical composition comprising a therapeutically effective amount of at least one compound selected from the group consisting of compounds as described above and optionally comprising a therapeutic agent selected from a chemotherapeutic or anti-proliferative agent, an immunomodulatory or immunosuppressive agent, or an anti-inflammatory agent.

Preferably, it is used for the prevention or treatment of neoplastic or immune conditions, especially related with the modulation or regulation of serine/threonine kinases, preferably selected from the group of Pim, HIPK, DYRK and CLK.

Preferably, for preventing or treating neoplastic conditions selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

The next subject of invention is a method for modulating or regulating serine/threonine kinases, preferably selected from the group of Pim, HIPK, DYRK and CLK kinases, wherein serine/threonine kinases are exposed to an effective amount of at least one compound of Formula I, an enantiomer thereof or a mixture of its enantiomers or pharmaceutically acceptable salts of compounds of Formula I or pharmaceutically acceptable prodrugs of compounds of Formula I, or pharmaceutically active metabolites of compounds of Formula I as described above.

Preferably, serine/threonine kinases, preferably kinases selected from the group of Pim, HIPK, DYRK and CLK kinases is in a subject with a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

The next subject of invention is a serine/threonine kinases modulating agent as described above, characterized in that serine/threonine kinases are preferably selected from the group of Pim, HIPK, DYRK and CLK kinases wherein serine/threonine kinases are exposed to an effective amount of at least one compound of Formula I, an enantiomer thereof or a mixture of its enantiomers or pharmaceutically acceptable salts of compounds of Formula I or pharmaceutically acceptable prodrugs of compounds of Formula I, or pharmaceutically active metabolites of compounds of Formula I as described above.

Preferably, serine/threonine kinases are preferably kinases selected from the group of Pim, HIPK, DYRK and CLK and their isoforms is in a subject with a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

The next subject of invention is a process for the preparation compound according to the above characterized in that the process comprise: reacting of a corresponding, unsubstituted or substituted 2,4,5,6,7-pentabromo-benzimidazole with a suitable amine at elevated temperature, wherein the reactive substituents X and/or Y are optionally protected with suitable protecting groups and wherein the resulting product is subjected to purification by crystallization or chromatography according to the reaction as shown in Scheme 1 below

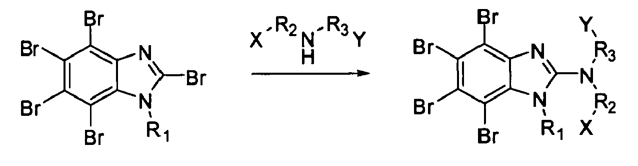

**Scheme 1:** synthetic route of production of a compound by reacting an unsubstituted or substituted 2,4,5,6,7-pentabromo-benzimidazole with a suitable amine.

While particular embodiments of the present disclosure have been illustrated as examples it is obvious to those skilled in the art that various additional changes can be introduced without departing from the spirit and scope of the disclosure. It is therefore intended to cover in the appended claims all such changes and modifications that are within spirit and scope of the disclosure.

Below, there are example embodiments of the present invention defined above.

### Examples

The following specific Examples are set forth to illustrate the invention and to aid in the understanding of the invention, and are not intended and should not be construed to limit in any way the invention set forth in the claims which follow thereafter.

The invention relates to compounds comprised by Formula I, with examples presented in Table 1 and their pharmaceutically acceptable salts, and pharmaceutically acceptable prodrugs.

Non-limiting examples of compounds comprised by Formula I are found in the Table 1.

**Table 1. Example list.**

| **Compound number** | **Name** | **Structure** |
|---|---|---|
| **11** | N-(3-aminopropyl)-4,5,6,7-tetrabromo-1H-benzo[d]imidazol-2-amine | 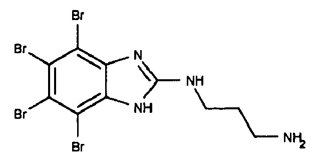 |
| **13** | 4,5,6,7-tetrabromo-N-(2-(piperidin-1-yl)ethyl)-1H-benzo[d]imidazol-2-amine | 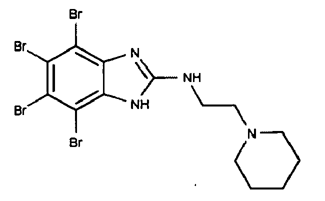 |
| **14** | 4,5,6,7-tetrabromo-N-(2-(pyrrolidin-1-yl)ethyl)-1H-benzo[d]imidazol-2-amine | 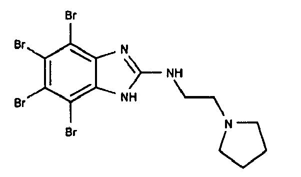 |
| **16** | 4,5,6,7-tetrabromo-N-(2-morpholinoethyl)-1H-benzo[d]imidazol-2-amine | 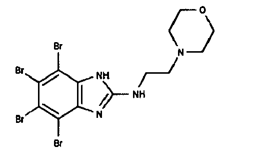 |
| **20** | 4,5,6,7-tetrabromo-2-(piperazin-1-yl)-1H-benzo[d]imidazole | 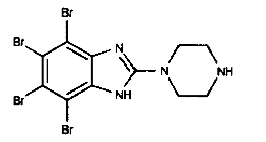 |
| **21** | 4,5,6,7-tetrabromo-2-(4-methylpiperazin-1-yl)-1H-benzo[d]imidazole | 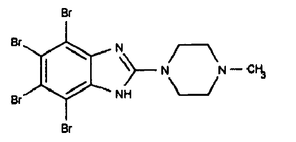 |
| **59** | 4,5,6,7-tetrabromo-N-(3-(piperidin-1-yl)propyl)-1H-benzo[d]imidazol-2-amine | 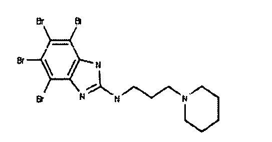 |
| **61** | N-(4-aminobutyl)-4,5,6,7-tetrabromo-1H-benzo[d]imidazol-2-amine | 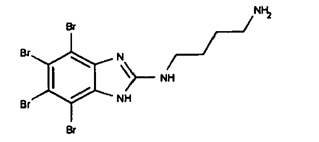 |
| **65** | N-(3-(1H-imidazol-1-y))propyl)-4,5,6,7-tetrabromo-1H-benzo[d]imidazol-2-amine | 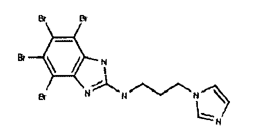 |
| **79** | 4,5,6,7-tetrabromo-N-phenyl-1H-benzo[d]imidazol-2-amine | 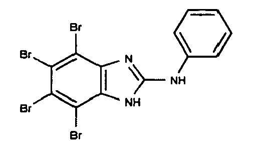 |
| **86** | N1-(4,5,6,7-tetrabromo-1H-benzo[d]imidazol-2-yl)benzene-1,4-diamine | 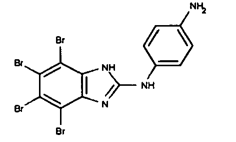 |
| **90** | 1-(4,5,6,7-tetrabromo-1H-benzo[d]imidazol-2-ylamino)-3-aminopropan-2-ol | 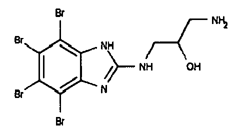 |

### Example 1. Synthesis of compound 11

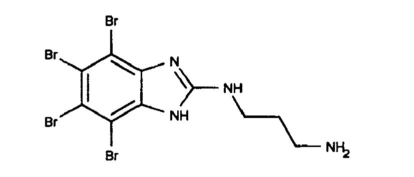

### N-(3-aminopropyl)-4,5,6,7-tetrabromo-1H-benzo[d]imidazol-2-amine

Pentabromobenzimidazole (500 mg, 0.98 mmo) and 1,3-diaminopropane (720 mg, 9.8 mmol) were heated in ethanol solution for 8 hours. Product was filtered off from the post reaction mixture and washed with methanol to give off white solid. Obtained product was 98% pure. MS (m/z): 506.8

### Example 2. Synthesis of compound 13

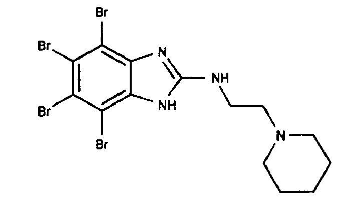

### 4,5,6,7-tetrabromo-N-(2-(piperidin-1-yl)ethyl)-1H-benzo[d]imidazol-2-amine

Pentabromobenzimidazole (600 mg, 1.17 mmo) and 1-(2-aminoethylpiperidine) (2.70 g, 21.07 mmol) were heated in ethanol solution for 8 hours. Product was filtered off from the post reaction mixture and washed with methanol to give off white solid. Obtained product was 99% pure. MS (m/z): 560.7

### Example 3. Synthesis of compound 14

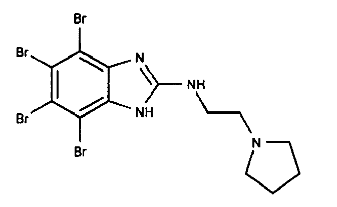

### 4,5,6,7-tetrabromo-N-(2-(pyrrolidin-1-yl)ethyl)-1H-benzo[d]imidazol-2-amine

Pentabromobenzimidazole (750 mg, 1.46 mmo) and 1-(2-aminoethyl)pyrrolidine (1.00 g, 8.78 mmol) were heated in ethanol solution for 8 hours. Product was filtered off from the post reaction mixture and washed with methanol to give off white solid. Obtained product was 94% pure. MS (m/z): 546.8

### Example 4. Synthesis of compound 16

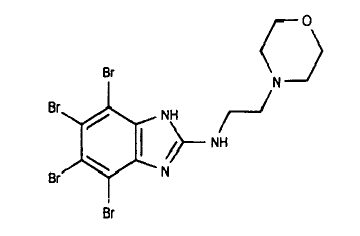

### 4,5,6,7-tetrabromo-N-(2-morpholinoethyl)-1H-benzo[d]imidazol-2-amine

Pentabromobenzimidazole (750 mg, 1.46 mmo) and 4-(2-aminoethyl)morpholine (2.48 g, 19.02 mmol) were heated in ethanol solution for 8 hours. Product was filtered off from the post reaction mixture and washed with methanol to give off white solid. Obtained product was 97.7% pure. MS (m/z): 562.8

### Example 5. Synthesis of compound 20

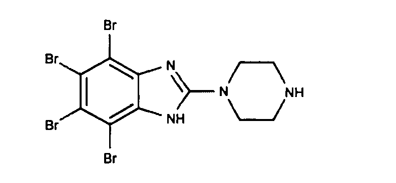

### 4,5,6,7-tetrabromo-2-(piperazin-1-yl)-1H-benzo[d]imidazole

Pentabromobenzimidazole (500 mg, 0.98 mmo) and piperazine (2.52 g, 29.26 mmol) were heated in ethanol solution for 8 hours. Product was filtered off from the post reaction mixture and washed with methanol to give off white solid. Obtained product was 96% pure. MS (m/z): 518.8

### Example 6. Synthesis of compound 21

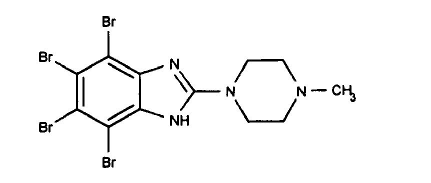

### 4,5,6,7-tetrabromo-N-(3-(piperidin-1-yl)propyl)-1H-benzo[d]imidazol-2-amine

Pentabromobenzimidazole (500 mg, 0.98 mmo) and 1-methylpiperazine (1 g, 10.7 mmol) were heated in ethanol solution for 16 hours. Product was filtered off from the post reaction mixture and washed with methanol to give off white solid. Obtained product was 98.4% pure. MS (m/z): 532.8

### Example 7. Synthesis of compound 59

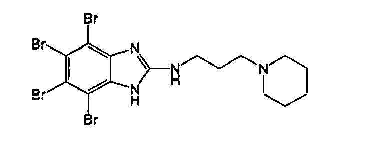

### 4,5,6,7-tetrabromo-N-(3-(piperidin-1-yl)propyl)-1H-benzo[d]imidazol-2-amine

Pentabromobenzimidazole (500 mg, 0.98 mmo) and 3-(piperidin-1-yl)propan-1-amine (1.39 g, 9.75mmol) were heated in ethanol solution for 16 hours. Product was filtered off from the post reaction mixture and washed with methanol to give off white solid. Obtained product was 97.3% pure. MS (m/z): 574.7

### Example 8. Synthesis of compound 61

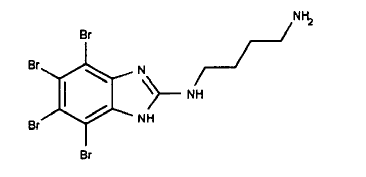

### N-(4-aminobutyl)-4,5,6,7-tetrabromo-1H-benzo[d]imidazol-2-amine

Pentabromobenzimidazole (500 mg, 0.98 mmo) and 1,4-diaminobutane (1.72 g, 19:51 mmol) were heated in ethanol solution for 10 hours. Product was isolated by means of flash chromatography (silica gel; methylene chloride:methanol:aqueous ammonia = 95:5:0.2) Obtained product was 94% pure. MS (m/z): 520.8

### Example 9. Synthesis of compound 65

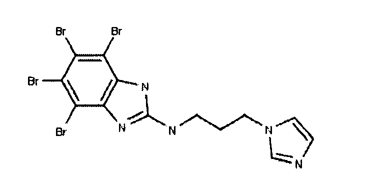

### N-(3-(1H-imidazol-1-yl)propyl)-4,5,6,7-tetrabromo-1H-benzo[d]imidazol-2-amine

Pentabromobenzimidazole (500 mg, 0.98 mmo) and 1-(3-diaminopropyl)imidazole (1.83 g, 14.63 mmol) were heated in ethanol solution for 12 hours. Product was isolated by means of flash chromatography (silica gel; chloroform:methanol:aqueous ammonia=8:2:0.2) Obtained product was 98% pure. MS (m/z): 557.7

### Example 10. Synthesis of compound 79

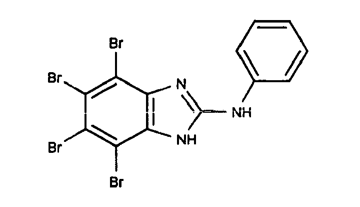

### 4,5,6,7-tetrabromo-N-phenyl-1H-benzo[d]imidazol-2-amine

Pentabromobenzimidazole (1000 mg, 1.95 mmo) and aniline (1.82 g, 19.51 mmol) were heated in ethanol solution for 11 hours. Product was filtered off from the post reaction mixture and washed with methanol to give off white solid. Obtained product was 97% pure. MS (m/z): 525.7

### Example 11. Synthesis of compound 86

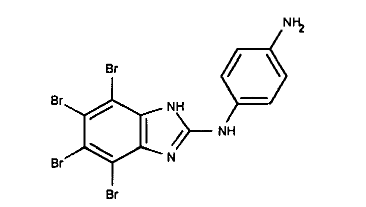

### N1-(4,5,6,7-tetrabromo-1H-benzo[d]imidazol-2-yl)benzene-1,4-diamine

Pentabromobenzimidazole (1000 mg, 1.95 mmo) and 1,4-diaminobenzene (2.14 g, 19.51 mmol) were heated in ethanol solution for 10hours. Product was isolated by means of flash chromatography (silica gel; chloroform:methanol = 9:1) Obtained product was 99% pure. MS (m/z): 540.5

### Example 12. Synthesis of compound 90

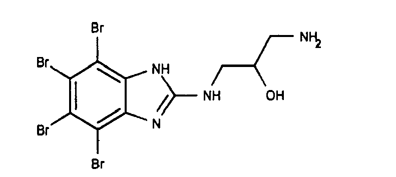

### 1-(4,5,6,7-tetrabromo-1H-benzo[d]imidazol-2-ylamino)-3-aminopropan-2-ol

Pentabromobenzimidazole (1000 mg, 1.95 mmo) and 1,3-diamino-2-propanol (1.76 g, 19.51 mmol) were heated in ethanol solution for 10 hours. Product was isolated by means of flash chromatography (silica gel; chloroform:methanol:aqueous amonia = 1:9:0.2) Obtained product was 99% pure. MS (m/z): 522.6

### Example 13. Growth inhibition test on human chronic myelocytic leukemia K562 cells - results summarized in Table 2

Ten thousand cells of human chronic myelocytic leukemia K562 were inoculated into each well of a 96-well microplate (manufactured by Coming Corp.) using Iscove's MDM medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 l MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37° C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 14. Growth inhibition test on human prostate adenocarcinoma PC3 cells - results summarized in Table 2

Two thousand cells of human prostate adenocarcinoma PC3 were inoculated into each well of a 96-well microplate (manufactured by Coming Corp.), and using F12K (Ham's F12:RPMI 8226 1: 1) medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37° C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 15. Growth inhibition test on human prostate carcinoma DU145 cells - results summarized in Table 2

Two thousand cells of human prostate carcinoma DU145 were inoculated into each well of a 96-well microplate (manufactured by Coming Corp.), and using DMEM medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, innersalt assay (CellTiter96® AQueous One Solution Cell Proliferation Assay, Promega) was added to each well, and culturing was performed in 5% carbon dioxide at 37° C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 16: Growth inhibition test on human breast adenocarcinoma MCF7 cells - results summarized in Table 2

Two thousand cells of human breast adenocarcinoma MCF7 were inoculated into each well of a 96-well microplate (manufactured by Coming Corp.), and using DMEM medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 17: Growth inhibition test on human colorectal adenocarcinoma SW480 cells - results summarized in Table 2

Two thousand cells of human colorectal adenocarcinoma SW480 were inoculated into each well of a 96-well microplate (manufactured by Coming Corp.), and using DMEM/ Ham's F121:1 medium (culture medium) containing 5% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 18: Growth inhibition test on human chronic myelogenous leukemia MEG-01 cells - results summarized in Table 2

Ten thousand cells of human chronic myelogenous leukemia MEG-01 were inoculated into each well of a 96-well microplate (manufactured by Coming Corp.) using RPMI 8226 medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 19: Growth inhibition test on human acute promyelocytic leukemia HL-60 cells - results summarized in Table 2

Ten thousand cells of human acute promyelocytic leukemia HL-60 were inoculated into each well of a 96-well microplate (manufactured by Coming Corp.) using RPMI 8226 medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 20: Growth inhibition test on human acute myeloid leukemia MOLM-13 cells - results summarized in Table 2

Ten thousand cells of acute myeloid leukemia MOLM-13 were inoculated into each well of a 96-well microplate (manufactured by Coming Corp.) using RPMI 8226 medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 21: Growth inhibition test on human myelomonocytic, biphenotypic leukemia MV4-11 cells - results summarized in Table 2

Ten thousand cells of human myelomonocytic, biphenotypic leukemia MV4-11 were inoculated into each well of a 96-well microplate (manufactured by Coming Corp.)using Iscove's MDM medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

### Example 22: Growth inhibition test on human acute lymphocytic leukemia E6.1 Jurkat cells - results summarized in Table 2

Twenty thousand cells of human acute lumphocytic leukemia E6.1 Jurkat were inoculated into each well of a 96-well microplate (manufactured by Coming Corp.) using RPMI 8226 medium (culture medium) containing 10% fetal calf serum (FCS). The same day, a dimethyl sulfoxide (DMSO) solution of each test compound prepared in a concentration of 10 mmol/L was further diluted with DMSO to the desired concentrations (0.1, 0.5, 1, 2.5, 5 and 10 micromol/L), and the diluted solution was added to each well. The individual wells were further cultured in 5% carbon dioxide at 37°C for 72 hours. After completion of the culture, 10 µl MTS (3-(4,5-Dimethyl-2-thiazolyl)-5-(carboxymethoxyphenyl)-2-(4-sulfophenyl)-2H-tetrazolium, inner salt assay (CellTiter96® AQueous One Solution Cell Proliferation Assay, Promega) was added to each well, and culturing was performed in 5% carbon dioxide at 37°C for 2 hours. Using a microplate spectrophotometer (Synergy 2 multi-mode microplate reader (BioTek)), the absorbance of each well was measured at 490 nm. The value for cells not incorporated with a test compound was designated as 100%. By comparing these values with the absorbance difference obtained at the well in which each test compound was added, the cell viability (% viability) after treatment with the test compound was calculated.

Whereas previously published tetrabromobenzimidazole derivatives were shown to have a cytotoxic activity on neoplastic cell lines *in vitro,* we found that the general cytotoxic activity of these compounds, exemplified by DMAT, TBB, TBI, K66 and K64 compounds (presented in Pagano *et al.* 2004 and 2008), defined as a compound concentration that results in death of 50% of the cell, is above 10 µM for majority of the tested cancer cell lines (summary in Table 2). For this reason we have qualified these compounds as moderately cytotoxic agents. In contrast to the previously published examples of tetrabromobenzimidazoles, new derivatives presented in the current application are more potent cytotoxic agents, as the ED50 values on at least 5 different neoplastic cell lines, exemplified in Table 2, of both hematologic and solid tumor origin are below 10 µM.

**Table 2. Anticancer activity of new derivatives (see Table 1) in in vitro cultured cancer cell lines**

| **Cytotoxicity in MTS assay (µM)** | | | | | | | | | | |
|---|---|---|---|---|---|---|---|---|---|---|
| **Cell line** | K562 | PC-3 | DU145 | MCF-7 | SW480 | MEG01 | HL-60 | MOLM13 | MV-4-11 | Jurkat |
| **Compound number** | | | | | | | | | | |
| **11** | 7,48 | 7,32 | 7,62 | 5,34 | 3,46 | 7,85 | 6,12 | 1,37 | 1,78 | 1,75 |
| **13** | 3,72 | 5,19 | 4,1 | 3,65 | 1,08 | 4,37 | 3,29 | 2,18 | 3,16 | 1,34 |
| **14** | 3,59 | 5,47 | 3,14 | 7,76 | 1,53 | 3,35 | 2,04 | 1,45 | 1,29 | 2,65 |
| **16** | | >10 | 8,64 | >10 | 9,68 | | >10 | 4,66 | 2,17 | 2,46 |
| **20** | 7,25 | 6,61 | 9,27 | | 4,91 | 4,49 | 7,88 | 1,59 | 0,79 | 2,19 |
| **21** | 7,99 | >10 | 9,54 | >10 | 5,98 | | >10 | 1,61 | 3,78 | 2,63 |
| **59** | | | | | | | | 3,51 | 2,81 | 4,41 |
| **61** | | | | | | | | 5,95 | 4,43 | 7,92 |
| **65** | | >10 | 7,72 | | 6,92 | | | 6,88 | >10 | >10 |
| **79** | | | | | | | | 5,96 | 4,4 | 9,06 |
| **86** | | | | | | | | 8,81 | 5,81 | 8,27 |
| **90** | | | | | | | | 6,91 | 2,12 | 5,78 |
| | | | | | | | | | | |
| **DMAT** | >10 | >10 | >10 | >10 | | 8,91 | >10 | | 3,15 | 8,18 |
| **TBB** | >10 | >10 | >10 | | >10 | >10 | | >10 | >10 | >10 |
| **TBI** | >10 | >10 | >10 | | 7,21 | >10 | | 9,72 | >10 | >10 |
| **K66** | >10 | >10 | >10 | | >10 | >10 | | >10 | >10 | >10 |
| **K64** | >10 | >10 | >10 | | 9,13 | >10 | | >10 | >10 | >10 |

### Example 23: Radiometric kinase assay for Pim kinases and CK2 kinase - results summarized in Table 4

Kinase assays were performed at Reaction Biology Corporation (www.reactionbiology.com, Malvern PA) using the "HotSpot" assay platform. This assay platform is based on a radiometric filtration binding assay and is considered as the "gold standard" to which non-radiometric methods are compared. In this assay format, the kinase reaction is performed in the presence of 32 P- γ -ATP or 33 P- γ -ATP, followed by binding of the final radioisotope labeled peptide product to filters after which unreacted phosphate is washed away and the levels of phosphorylated peptide are measured. Reaction conditions are presented in Table 3.

**Table 1. Reaction conditions for radiometric kinase assay.**

| **Kinase** | **Peptide substrate sequence** | **Peptide concentration** | **ATP concentration** |
|---|---|---|---|
| **Pim-1** | KKRNRTLTK | 5 µM | 1 µM |
| **Pim-2** | RSRHSSYPAGT | 5 µM | 1 µM |
| **Pim-3** | RSRHSSYPAGT | 20 µM | 1 µM |
| **CK2α** | RRRDDDSDDD | 20 µM | 1 µM |
| **CLK1** | Myelin Basic Protein | 10 µM | 1 µM |

In the prior art document by Pagano *et al.* 2008, certain tetrabromobenzimidazoles with both Pim-1 and CK2 inhibitor activity were described. However, whereas the described compounds show potent CK2 kinase inhibition in vitro with IC50 values below 100 nM, none of the presented compounds showed an IC50 value below 100 nM for either Pim-1 or Pim-2 kinase and can be regarded as a potent Pim kinase inhibitor. Also, none of the compounds showed an at least 10-fold selectivity for inhibition any of the Pim kinases over CK2. In contrast, certain embodiments of the current application provide examples of compounds with improved potency in inhibition of any of the Pim kinase and at least 10-fold specificity in inhibition of Pim-1, Pim-2 or Pim-3 kinase over the CK2α kinase.

**Table 2. IC50 values for inhibition of Pim kinases and CK2alpha kinase for new derivatives and their selectivity in comparison to prior art compound examples.**

| | Inhibitory activity (IC50 in nM) in radiometric cell free assay | | | | | Selectivity for Pim-1 over CK2α | Selectivity for Pim-2 over CK2α | Selectivity for Pim-3 over CK2α |
|---|---|---|---|---|---|---|---|---|
| Kinase | Pim-1 | Pim-2 | Pim-3 | CK2α | CLK1 | | | |
| Compound name | | | | | | | | |
| 11 | 4,8 | 98,2 | 23,3 | 1016,0 | 177,0 | 211,7 | 10,3 | 43,6 |
| 13 | 23,1 | 896,9 | 60,7 | 2263,0 | 593,9 | 98,2 | 2,5 | 37,3 |
| 14 | 43,8 | 618,4 | 28,2 | 833,1 | | 19,0 | 1,3 | 29,6 |
| 16 | 67,7 | 3008,0 | 114,5 | 6876,0 | | 101,6 | 2,3 | 60,1 |
| 20 | 76,9 | 325,9 | 42,0 | 131,9 | | 1,7 | 0,4 | 3,1 |
| 21 | 66,1 | 1693,0 | 84,1 | 1826,0 | | 27,6 | 1,1 | 21,7 |
| 59 | 88,0 | 1492,0 | 188,7 | 854,8 | | 9,7 | 0,6 | 4,5 |
| 61 | 5,2 | 198,7 | 20,2 | 385,7 | | 74,2 | 1,9 | 19,1 |
| 65 | 87,1 | 823,2 | 15,8 | 447,5 | | 5,1 | 0,5 | 28,3 |
| 79 | 1513,0 | >20000 | 2964,0 | 1960,0 | | 1,3 | >0,1 | 0,7 |
| 86 | 546,6 | 1546,0 | 680,0 | 733,9 | | 1,3 | 0,5 | 1,1 |
| 90 | 9,1 | 90,3 | 7,6 | 109,8 | | 12,0 | 1,2 | 14,4 |
| | | | | | | | | |
| DMAT | 64,6 | 2464,0 | 87,3 | 60,0 | 210,6 | 0,9 | 0,0 | 0,7 |
| TBB | 1839,5 | >20000 | 269,2 | 629,3 | | 0,3 | >0,1 | 2,3 |
| TBI | 1674,0 | >20000 | 2945,0 | 217,7 | | 0,1 | >0,1 | 0,1 |
| K66 | 194,2 | 954,4 | 96,3 | 206,3 | | 1,1 | 0,2 | 2,1 |
| K64 | 308,0 | 870,9 | 212,0 | 636,7 | | 2,1 | 0,7 | 3,0 |

### Example 24: KinomeScan Max kinase binding inhibition panel - results summarized in Table 5

Kinase panel profiling was performed at Ambit Kinomescan (http://www.ambitbio.com/technology/) using the "KINOMEscan™ Max" assay platform, a high-throughput method for screening small molecule compounds against large numbers of human kinases. KINOMEscan™ is based on a competition binding assay that quantitatively measures the ability of a compound to compete with an immobilized, active-site directed ligand. The assay is performed by combining three components: DNA-tagged kinase; immobilized ligand; and a test compound. The ability of the test compound to compete with the immobilized ligand is measured via quantitative PCR of the DNA tag. For a description of Ambit's assay protocol, see Fabian et al. A small molecule-kinase interaction map for clinical kinase inhibitors. Nat. Biotechnol. 23, 329-336 (2005).

Selectivity of kinase inhibition is one of the key parameters in therapeutic use of this class of compounds. Off-target inhibition could be one of the major safety and toxicity issues in drug development and use of the compounds for treatment of various disease conditions. For this reason, development of selective kinase inhibitors is desired, to limit possible adverse effects. Therefore compounds disclosed in the application were tested on a panel of 440 kinases to determine specificity in PIM kinase inhibition. Table 5 provides selectivity data for representative compound N-(3-aminopropyl)-4,5,6,7-tetrabromo-1H-benzo[d]imidazol-2-amine (compound 11). As indicated in the Table 5 when tested at 1 µM concentration N-(3-aminopropyl)-4,5,6,7-tetrabromo-1H-benzo[d]imidazol-2-amine inhibited substrate biding of only 12 kinases (2% of the whole panel) >90%, whereas for over 93% of the tested kinases in the panel, substrate binding was basically not affected by compound 11. Pim kinases, but also HIPK, DYRK and CLK kinases and FTL3 mutants were identified as the primary targets for inhibition by compound 11 and selectivity for Pim kinases over other kinases was further confirmed in radiometric kinase assays.

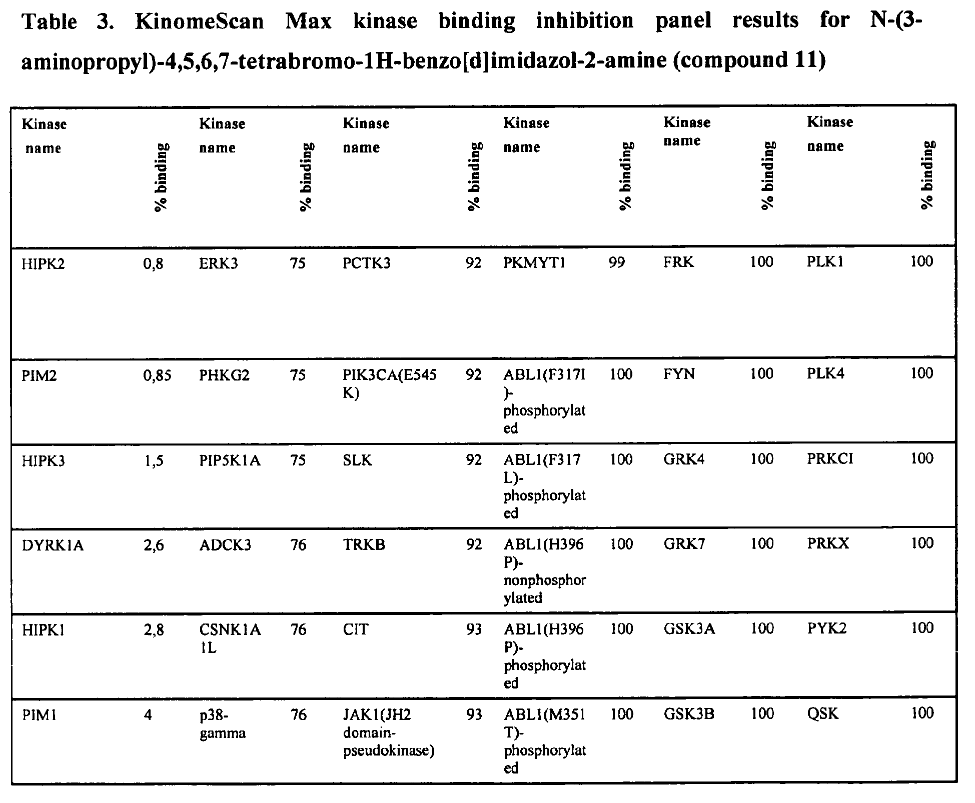

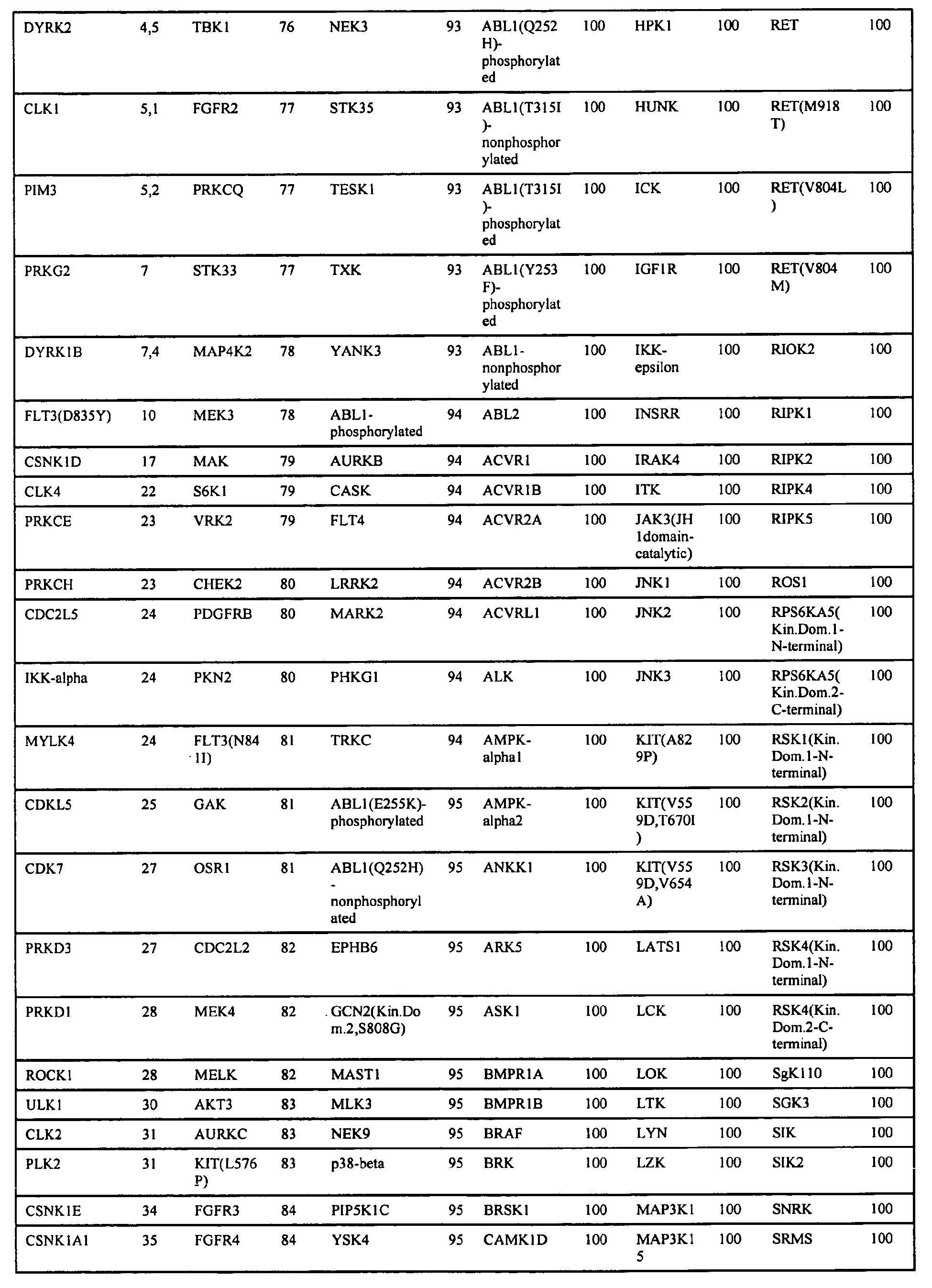

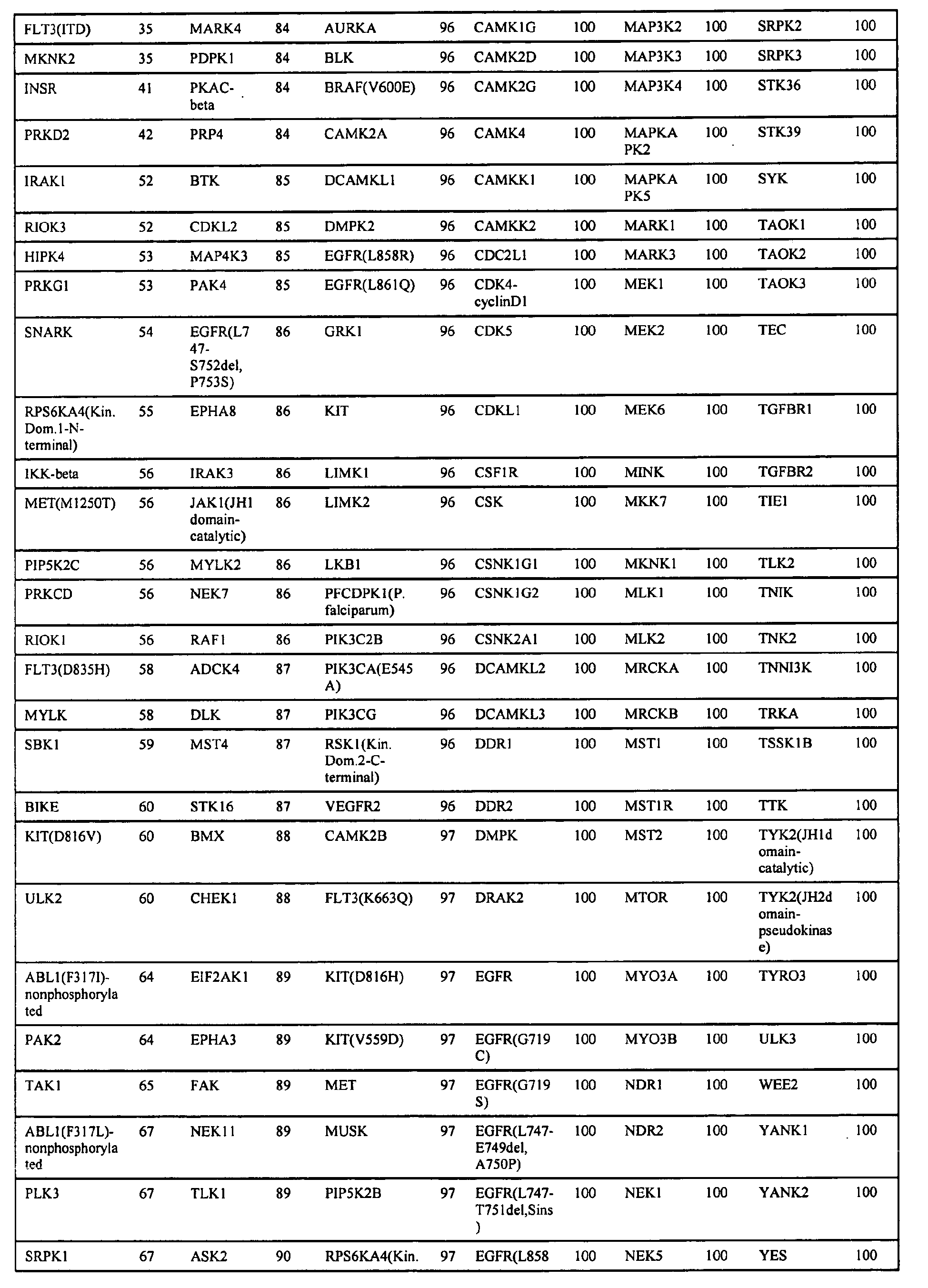

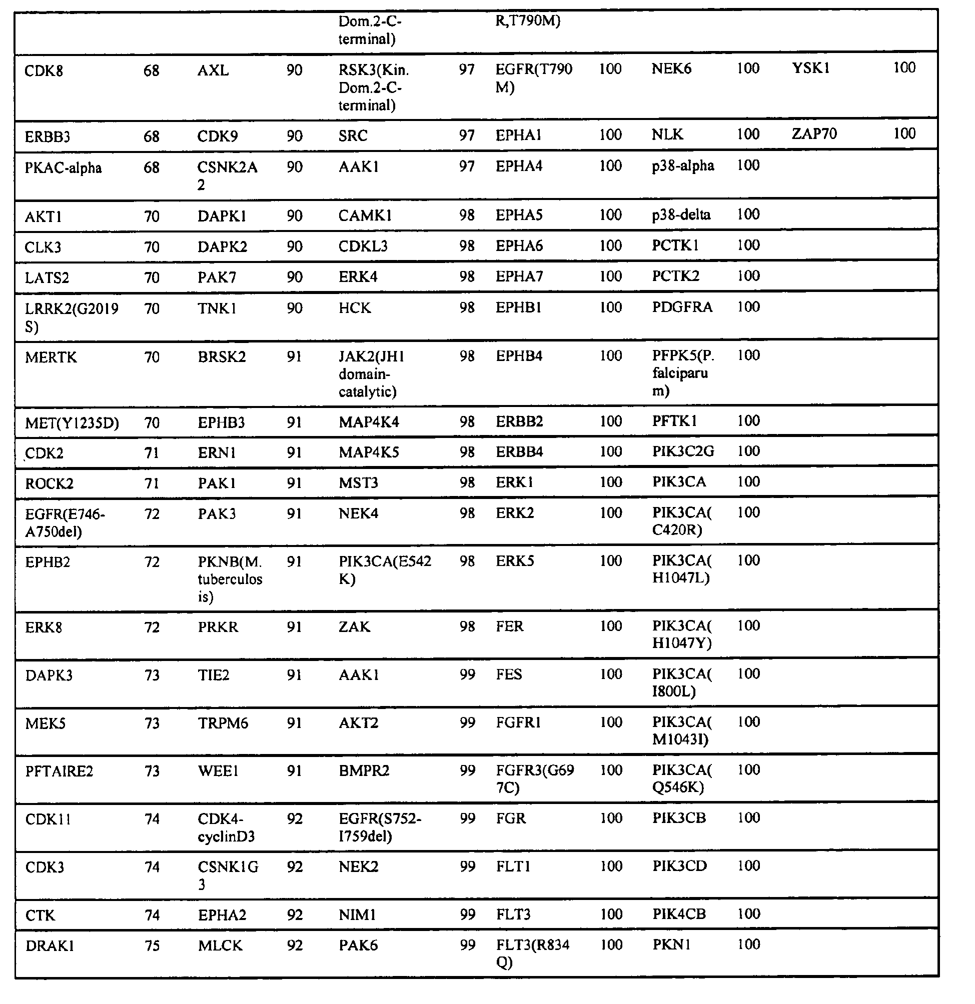

### Example 25: Solubility comparison of the compounds in pH 7.4 - results summarized in Table 6

Compound stock solutions were prepared in DMSO to final concentrations of 1000 mM. For each compound studied, 12 solutions were prepared to cover concentrations from 0.001 to 1 mM. As a solvent 0.2 mM phosphate buffer (pH 7.4) was used. The final concentration of DMSO in the solutions was 1% (v/v). The solutions were left mixing for 24 hours at 37°C, 350 rpm and following incubation centrifuged for 5 minutes at 14500 rpm. The samples were then analyzed by RP HPLC using a C18 column and 0.2% solution of formic acid in water/acetonitrile mobile phase. For low concentration solutions the HPLC signal dependence on compound concentration is linear and reaches plateau for higher concentrations. The solubility was determined as the point as the point at which the concentration curve reaches plateau.

Examples of the results for chosen compounds are shown in Table 6. In general, tetrabromobenzimidazoles are poorly soluble compounds, what renders their chances for successful therapeutic administration is commonly used routes of administration and standard conditions. In contrast to previously published tetrabromobenzimidazoles exemplified by DMAT, certain compounds provided in the application show a markedly increase in water solubility between 2 to 37 fold. As solubility is one of the key parameters that influences compound pharmacokinetics, permeability and therefore also activity *in vitro* and *in vivo,* improved solubility of the selected compounds is a surprising feature that can lead to improved efficacy in treating Pim kinase associated disease conditions.

**Table 4. Solubility of the compounds in pH 7.4.**

| **Compound No.** | Solubility [mM] in phosphate buffer pH 7.4 |
|---|---|
| **DMAT** | <0,01 |
| **11** | 0,37 |
| **14** | 0,031 |
| **16** | 0,023 |
| **20** | 0,081 |
| **21** | <0,01 |
| **90** | 0,072 |

## Claims

1. A compound of formula (I):
or an enantiomer thereof or a mixture of its enantiomers or its pharmaceutically acceptable salt, or pharmaceutically acceptable prodrugs or pharmaceutically active metabolites, wherein:
R₁ is selected from a group consisting of: hydrogen atom, substituted or unsubstituted alkyl or cycloalkyl, substituted or unsubstituted alkenyl or alkynyl, substituted or unsubstituted aryl, substituted or unsubstituted heteroalkyl or heteroaryl;
R₂ and R₃ are independently selected from a group consisting of: hydrogen atom, substituted or unsubstituted hydrocarbon chain, substituted or unsubstituted cycloalkyl, substituted or unsubstituted aryl, substituted or unsubstituted heteroaryl, substituted or unsubstituted heterocycloalkyl;
X and Y are independently selected from a hydrogen atom, substituted or unsubstituted heteroaryl, guanidinyl , -NH₂, -NHR0 or N(R0)₂, where R0 represents a substituted or unsubstituted alkyl, aryl or heteroaryl and/or wherein X and Y can also represent a substituted or unsubstituted heterocyclic moiety having 3-10 atoms wherein at least one of the ring atoms is nitrogen
or
R₂ and R₃ are connected to a substituted or unsubstituted heterocycloalkyl, wherein the following structures are excluded:
2-amino-4,5,6,7-tetrabromo-1H-benzimidazole;
2-methylamino-4,5,6,7-tetrabromo-1H-benzimidazole;
2-dimethylamino-4,5,6,7-tetrabromo-1H-benzimidazole;
2-ethanolamino-4,5,6,7-tetrabromo-1H-benzimidazole;
2-isopropylamino-4,5,6,7-tetrabromo-1H-benzimidazole;
2-(2-hydroxypropylamino)-4,5,6,7-tetrabromo-1H-benzimidazole;
2-(3-hydroxypropylamino)-4,5,6,7-tetrabromo-1H-benzimidazole;
2-(2-dimethylaminoethylamino)-4,5,6,7-tetrabromo-1H-benzimidazole;
2-dimethylamino-4,5,6,7-tetrabromo-1-methyl-benzimidazole;
2-isopropylamino-4,5,6,7-tetrabromo-1methyl-benzimidazole;
2-(methyl(4,5,6,7-tetrabromo-1-methyl-1H-benzo[d]imidazol-2-yl)amino)ethanol;
(2-dimethylamino-4,5,6,7-tetrabromobenzoimidazol-1-yl)-acetic acid ethyl ester;
(2-dimethylamino-4,5,6,7-tetrabromobenzoimidazol-1-yl)-acetic acid.

2. The compound according to claim 1, **characterised in that**
R₁ is selected among hydrogen atom, substituted or unsubstituted alkyl, cycloalkyl, alkenyl or alkynyl;
R₂ is H, and R₃ is a C₂-C₅ alkyl substituted with Y selected from: -NH₂, -NH(C=NH)-NH₂, -NHR0, -N(R0)₂, wherein R0 represents a substituted or unsubstituted alkyl, aryl or heteroaryl.

3. The compound according to claim 1, **characterised in that** the compound is preferably selected from:
N 1-(4,5,6,7-tetrabromo-1-methyl-1H-benzo[d] imidazol-2-yl)ethane-1,2-diamine;
N1-(4,5,6,7-tetrabromo-1H-benzo[d]imidazol-2-yl)propane-1,3-diamine;
N1-(4,5,6,7-tetrabromo-1-methyl-1H-benzo[d]imidazol-2-yl)propane-1,3-diamine;
N1-(4,5,6,7-tetrabromo-1-isopropyl-1H-benzo[d]imidazol-2-yl)propane-1,3-diamine;
1-(2-(4,5,6,7-tetrabromo-1H-benzo[d]imidazol-2-ylamino)ethyl)guanidine;
1-(3-(4,5,6,7-tetrabromo-1H-benzo[d]imidazol-2-ylamino)propyl)guanidine;
N1,N1-dimethyl-N3-(4,5,6,7-tetrabromo-1H-benzo[d]imidazol-2-yl)propane-1,3-diamine;
N1,N1-dimethyl-N3-(4,5,6,7-tetrabromo-1-methyl-1H-benzo[d]imidazol-2-yl)propane-1,3-diamine;
N1-(4,5,6,7-tetrabromo-1H-benzo[d]imidazol-2-yl)butane-1,4-diamine;
N1-(4,5,6,7-tetrabromo-1-methyl-1H-benzo[d]imidazol-2-yl)butane-1,4-diamine;
N 1-(4,5,6,7-tetrabromo-1-isopropyl-1H-benzo[d]imidazol-2-yl)butane-1,4-diamine.

4. The compound according to claim 1, **characterised in that** R₁ is selected among hydrogen atom, substituted or unsubstituted alkyl, cycloalkyl, alkenyl or alkynyl;
R₂ is H, and R3 is a C₂-C₅ alkyl substituted with Y selected from a substituted or unsubstituted heterocyclic moiety having 3-10 atoms, preferentially the following:

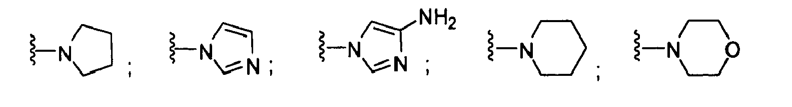

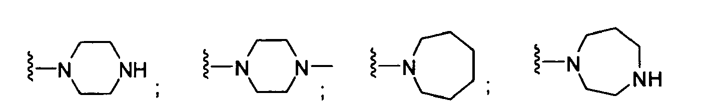

5. The compound according to claim 4, **characterised in that** the compound is preferably selected from:
4,5,6,7-tetrabromo-N-(2-(pyrrolidin-1-yl)ethyl)-1H-benzo[d]imidazol-2-amine;
N-(2-(1H-imidazol-1-yl)ethyl)-4,5,6,7-tetrabromo-1H-benzo[d]imidazol-2-amine;
4,5,6,7-tetrabromo-N-(2-(piperidin-1-yl)ethyl)-1H-benzo[d]imidazol-2-amine;
4,5,6,7-tetrabromo-N-(2-morpholinoethyl)-1H-benzo[d]imidazol-2-amine;
4,5,6,7-tetrabromo-N-(2-(piperazin-1-yl)ethyl)-1H-benzo[d]imidazol-2-amine;
N-(2-(azepan-1-yl)ethyl)-4,5,6,7-tetrabromo-1H-benzo[d]imidazol-2-amine;
N-(2-(azepan-1-yl)ethyl)-4,5,6,7-tetrabromo-1-methyl-1H-benzo[d]imidazol-2-amine;
N-(2-(1,4-diazepan-1-yt)ethyl)-4,5,6,7-tetrabromo-1H-benzo[d]imidazol-2-amine;
N-(2-(1,4-diazepan-1-yl)ethyl)-4,5,6,7-tetrabromo-1-methyl-1H-benzo[d]imidazol-2-amine;
N-(2-(1,4-diazepan-1-yl)ethyl)-4,5,6,7-tetrabromo-1-isopropyl-1H-benzo[d]imidazol-2-amine.

6. The compound according to claim claim 1, **characterised in that**
R₁ is selected from hydrogen atom, substituted or unsubstituted alkyl, cycloalkyl, alkenyl or alkynyl;
R₂ and R₃ are independently selected from C₂-₅ alkyl optionally substituted with X and/or Y selected from: -NH₂, -NH(C=NH)-NH₂, -NHR0, -N(R0)₂, where R0 represents a substituted or unsubstituted alkyl , aryl or heteroaryl, and wherein the compound is preferably selected from:
N1-ethyl-N1-(4,5,6,7-tetrabromo-1H-benzo[d]imidazol-2-yl)ethane-1,2-diamine;
N 1-(2-aminoethyl)-N1-(4,5,6,7-tetrabromo-1H-benzo[d]imidazol-2-yl)ethane-1,2-diamine;
N 1-(2-aminoethyl)-N1-(4,5,6,7-tetrabromo-1-methyl-1H-benzo[d]imidazol-2-yl)ethane-1,2-diamine;
N1-ethyl-N1-(4,5,6,7-tetrabromo-1H-benzo[d]imidazol-2-yl)propane-1,3-diamine;
N 1-(3-aminopropyl)-N1-(4,5,6,7-tetrabromo-1H-benzo[d]imidazol-2-yl)propane-1,3-diamine;
N 1-(3-aminopropyl)-N3,N3-dimethyl-N1-(4,5,6,7-tetrabromo-1H-benzo[d]imidazol-2-yl)propane-1,3-diamine;
N1-(3-aminopropyl)-N3,N3-dimethyl-N1-(4,5,6,7-tetrabromo-1-methyl-1H-benzo[d]imidazol-2-yl)propane-1,3-diamine;
1-(3-((3-(dimethylamino)propyl)(4,5,6,7-tetrabromo-1H-benzo[d]imidazol-2-yl)amino)propyl)guanidine;
1-(3-((3-(dimethylamino)propyl)(4,5,6,7-tetrabromo-1-methyl-1H-benzo[d]imidazol-2-yl)amino)propyl)guanidine.

7. The compound according to claim 1, **characterised in that**
R₁ is selected from hydrogen atom, substituted or unsubstituted alkyl, cycloalkyl, alkenyl or alkynyl;
R₂ is H, and R₃ is selected from substituted or unsubstituted aryl or substituted or unsubstituted heteroaryl , and wherein the compound is preferably selected from:
4,5,6,7-tetrabromo-N-phenyl-1H-benzo[d]imidazol-2-amine;
4,5,6,7-tetrabromo-1-methyl-N-phenyl-1H-benzo[d]imidazol-2-amine;
N 1-(4,5,6,7-tetrabromo-1H-benzo[d]imidazol-2-yl)benzene-1,4-diamine;
N 1-(4,5,6,7-tetrabromo-1-methyl-1H-benzo[d]imidazol-2-yl)benzene-1,4-diamine;
N 1,N1-dimethyl-N4-(4,5,6,7-tetrabromo-1H-benzo[d]imidazol-2-yl)benzene-1,4-diamine;
N1,N1-dimethyl-N4-(4,5,6,7-tetrabromo-1-methyl-1H-benzo[d]imidazol-2-yl)benzene-1,4-diamine.

8. The compound according to claim 1, **characterised in that** R₁ is selected among hydrogen atom, substituted or unsubstituted alkyl, cycloalkyl, alkenyl or alkynyl, and R₂ and R₃ are connected to a substituted or unsubstituted heterocycloalkyl, and wherein the compound is preferably selected from:
4,5,6,7-tetrabromo-2-(pyrrolidin-1-yl)-1H-benzo[d]imidazole;
4,5,6,7-tetrabromo-2-(piperidin-1-yl)-1H-benzo[d]imidazole;
4-(4,5,6,7-tetrabromo-1H-benzo[d]imidazol-2-yl)morpholine;
4,5,6,7-tetrabromo-2-(piperazin-1-yl)-1H-benzo[d]imidazole;
4,5,6,7-tetrabromo-2-(4-methylpiperazin-1-yl)-1H-benzo[d]imidazole;
4,5,6,7-tetrabromo-1-methyl-2-(4-methylpiperazin-1-yl)-1H-benzo[d]imidazole;
4,5,6,7-tetrabromo-1-isopropyl-2-(4-methylpiperazin-1-yl)-1H-benzo[d]imidazole.

9. A compound according to any of the above claims 1 to 8, **characterized in that** pharmaceutically acceptable salt is selected preferably from the group consisting of hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, methanesulfonate, ethanesulfonate, benzensulfonate, p-toluenesulfonate and pamoate and the like.

10. A pharmaceutical composition, comprising a therapeutically effective amount of at least one compound selected from the group consisting of compounds as claimed in any one of claims 1 to 9 and optionally comprising a therapeutic agent selected from a chemotherapeutic or anti-proliferative agent, an immunomodulatory or immunosuppressive agent, or an anti-inflammatory agent.

11. A pharmaceutical composition, according to claim 10, **characterized in that** the therapeutically effective amount provided in the treatment is administered in an amount of about 0,01 to 1,000 mg/kg at least once a day for the duration of the treatment.

12. A pharmaceutical composition, according to claim 10, **characterized in that** the said composition optionally comprises a therapeutic agent selected from a chemotherapeutic or anti-proliferative agent, an immunomodulatory or immunosuppressive agent, or an anti-inflammatory agent

13. A pharmaceutical composition, according to claim 10 or 11, **characterized in that** said composition is administered parenterally, vaginally, rectally, transdermally, orally or through the otolaryngologal sphere.

14. A pharmaceutical composition, according to claim 10 or 11, **characterized in that** it further comprises a pharmaceutically acceptable carrier selected from the group consisting of flavoring agents, sweetener, lubricants, solubilizers, suspending agents, fillers, glidants, compression aides, binders, tablet-disintegrating agents, effervescing agent, wetting agent encapsulating materials, dyestuff, and mixtures thereof wherein carrier is chosen from solid or liquid carriers, whether sterile or not.

15. A pharmaceutical composition, according to claim 10 or 11, **characterized in that** said composition is a sterile solution or suspension suitable for parenteral administration, including but not limited to intramuscular, intraperitoneal, intravenous, intrathecal or subcutaneous injection or perfusion.

16. A pharmaceutical composition, according to claim 10 or 11, **characterized in that** suitable for oral administration either liquid or solid composition form, including pills, tablets, capsules, sachets, dragees, powders, granules, lozenges, powders for reconstitution, orally disintegrating tablet, films, osmotic controlled release capsule, elixir, emulsion, syrup, suspension, tincture, solutions or powder for inhalation and nebulization, or sublingual administration.

17. A pharmaceutical composition, according to claim 10 or 15, **characterized in that** said composition is a formulation for transdermal, rectal, vaginal administration, including ointments, creams, lotions, liniments, gels, paste, films, suppositories, enemas and pessaries.

18. A pharmaceutical composition, according to claim 10 or 11, **characterized in that** said composition is a formulation for administration through the eyes, ears and nose.

19. A pharmaceutical composition, according to claim 10 or 11, **characterized in that** the composition is an immediate, extended or slow- release formulation.

20. A pharmaceutical composition, according to claim 10 or 11, **characterized in that** the therapeutically effective amount is provided in the treatment of a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma,
lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

21. A pharmaceutical composition according to claim 10 or 11 for the prevention or treatment of neoplastic conditions, especially related with the modulation or regulation of serine/threonine kinases, preferably selected from the group of Pim, HIPK, DYRK and CLK kinases.

22. Use of the compound of Formula I for the preparation of a pharmaceutical composition comprising a therapeutically effective amount of at least one compound selected from the group consisting of compounds as claimed in any one of claims 1 to 9 and optionally comprising a therapeutic agent selected from a chemotherapeutic or anti-proliferative agent, an immunomodulatory or immunosuppressive agent, or an anti-inflammatory agent.

23. Use according to claim 22 for the prevention or treatment of neoplastic or immune conditions, especially related with the modulation or regulation of serine/threonine kinases, preferably selected from the group of Pim, HIPK, DYRK and CLK.

24. Use according to claim 22 or 23 for preventing or treating neoplastic conditions selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

25. A method for modulating or regulating serine/threonine kinases, preferably selected from the group of Pim, HIPK, DYRK and CLK kinases, wherein serine/threonine kinases are exposed to an effective amount of at least one compound of Formula I, an enantiomer thereof or a mixture of its enantiomers or pharmaceutically acceptable salts of compounds of Formula I or pharmaceutically acceptable prodrugs of compounds of Formula I, or pharmaceutically active metabolites of compounds of Formula I as claimed in any one of claims 1 to 9.

26. A method according to claim 25, **characterized in that** serine/threonine kinases, preferably kinases selected from the group of Pim, HIPK, DYRK and CLK kinases is in a subject with a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma, fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

27. A serine/threonine kinases modulating agent as claimed in any one of claims 1 to 9, **characterized in that** serine/threonine kinases are preferably selected from the group of Pim, HIPK, DYRK and CLK kinases wherein serine/threonine kinases are exposed to an effective amount of at least one compound of Formula I, an enantiomer thereof or a mixture of its enantiomers or pharmaceutically acceptable salts of compounds of Formula I or pharmaceutically acceptable prodrugs of compounds of Formula I, or pharmaceutically active metabolites of compounds of Formula I as claimed in any one of claims 1 to 9.

28. An agent according to claim 27, **characterized in that** serine/threonine kinases are preferably kinases selected from the group of Pim, HIPK, DYRK and CLK and their isoforms is in a subject with a disease, disorders or medical condition that is selected from the group consisting of myeloid leukemia both acute and chronic, acute lymphoblastic leukemia, chronic lymphocytic leukemia, hairy cell leukemia, myeloproliferative diseases, multiple myeloma, myelodysplastic syndrome, Hodgkin's disease, non-Hodgkin's lymphoma (malignant lymphoma); adenocarcinoma, lymphoma, leukemia of the kidney, Wilm's tumor, renal cell carcinoma, renal pelvis carcinoma, nephroma, teratoma, sarcoma of the kidney, squamous cell carcinoma, transitional cell carcinoma, adenocarcinoma of bladder and urethra, adenocarcinoma, sarcoma of the prostate, seminoma, teratoma, embryonal carcinoma, teratocarcinoma, choriocarcinoma, sarcoma, interstitial cell carcinoma, fibroma, fibroadenoma, adenomatoid tumors, lipoma of the testis; angiosarcoma, fibrosarcoma, rhabdomyosarcoma, liposarcoma, myxoma, rhabdomyoma, fibroma, lipoma and teratoma of the heart; astrocytoma, medulloblastoma, glioma, ependymoma, germinoma [pinealoma], glioblastorna multiform, oligodendroglioma, schwannoma, retinoblastoma, congenital tumors of the brain, neurofibroma, meningioma, glioma, sarcoma of the spinal cord, osteoma, hemangioma, granuloma, xanthoma, osteitis deformians of the skull, meningioma, meningiosarcoma, gliomatosis of the meninges; squamous cell, undifferentiated small cell, undifferentiated large cell, adenocarcinoma, alveolar carcinoma, bronchial adenoma, sarcoma, lymphoma, chondromatous hanlartoma, mesothelioma of the bronchus; adenocarcinoma, lymphoma, carcinoid tumors, Karposi's sarcoma, leiomyoma, hemangioma, lipoma, neurofibroma; fibromaof the small bowel, adenocarcinoma, tubular adenoma, villous adenoma, hamartoma, leiomyoma of the large bowel; squamous cell carcinoma, adenocarcinoma, leiomyosarcoma, lymphoma of the esophagus, carcinoma, lymphoma, leiomyosarcoma of the stomach, ductal adenocarcinoma, insulinoma, glucagonoma, gastrinoma, carcinoid tumors, vipoma of the pancreas; hepatocellular carcinoma, cholangiocarcinoma, hepatoblastoma, angiosarcoma, hepatocellular adenoma, hemangioma of the liver; osteogenic sarcoma, fibrosarcoma, malignant fibrous histiocytoma, chondrosarcoma, Ewing's sarcoma, malignant lymphoma such as reticulum cell sarcoma, multiple myeloma, malignant giant cell tumor chordoma, osteochronfroma such as osteocartilaginous exostoses, benign chondroma, chondroblastoma, chondromyxofibroma, osteoid osteoma and giant cell tumors; endometrial carcinoma, cervical carcinoma, pre-tumor cervical dysplasia, ovarian carcinoma such as serous cystadenocarcinoma, mucinous cystadenocarcinoma, unclassified carcinoma, granulosa-thecal cell tumors, Sertol/Leydig cell tumors, dysgerminoma, malignant teratoma of the ovary, squamous cell carcinoma, intraepithelial carcinoma, adenocarcinoma, fibrosarcoma, melanoma of the vulva, clear cell carcinoma, squamous cell carcinoma, botryoid sarcoma such as embryonal rhabdomyosarcoma of the vagina, fallopian tubes carcinoma), breast; and malignant melanoma, basal cell carcinoma, squamous cell carcinoma, Karposi's sarcoma, moles dysplastic nevi, lipoma, angioma, dermatofibroma, keloids, bone marrow transplant rejection, rheumatoid arthritis, psoriasis, type I diabetes mellitus and multiple sclerosis.

29. Process for the preparation of a compound according to any one of claims 1 to 9 **characterised in that** the process comprise: reacting of a corresponding, unsubstituted or substituted 2,4,5,6,7-pentabromo-benzimidazole with a suitable amine at elevated temperature, wherein the reactive substituents X and/or Y are optionally protected with suitable protecting groups and wherein the resulting product is subjected to purification by crystallization or chromatography according to the reaction
